(19) Europäisches Patentamt
European Patent Office
Office européen des brevets

(11) EP 0 589 484 B1

(12) EUROPEAN PATENT SPECIFICATION

(45) Date of publication and mention of the grant of the patent:
**16.10.1996 Bulletin 1996/42**

(21) Application number: **93115918.0**

(22) Date of filing: **13.02.1990**

(51) Int Cl.[6]: **C07C 217/10**, A61K 31/13, C07D 295/088, A61K 31/33, C07D 207/27, C07D 207/16, C07D 207/08, C07D 309/12, C07D 213/74, C07D 239/42

# (54) 1,2-Ethanediol derivative and salt thereof, process for producing the same, and cerebral function-improving agent comprising the same

1,2-Ethandiolderivate und ihre Salze, Verfahren zu ihrer Herstellung und sie enthaltende gehirnfunktionsverbessernde Mittel

Dérivés d'éthanediol-1,2 et leurs sels, procédé de leur préparation et agents rétablissant la fonction cérébrale les contenant

(84) Designated Contracting States:
**AT CH DE DK GB LI NL SE**

(30) Priority: **14.02.1989 JP 32714/89**
**20.03.1989 JP 68958/89**
**26.04.1989 JP 106187/89**
**05.02.1990 JP 24501/90**
**05.02.1990 JP 24502/90**
**05.02.1990 JP 24503/90**

(43) Date of publication of application:
**30.03.1994 Bulletin 1994/13**

(62) Application number of earlier application in accordance with Art. 76 EPC: **90102829.0**

(73) Proprietor: **TOYAMA CHEMICAL CO., LTD.**
**Tokyo 160 (JP)**

(72) Inventors:
- **Ono, Satoshi**
  **Toyama-shi (JP)**
- **Yamafuji, Tetsuo**
  **Nei-gun, Toyama-ken (JP)**
- **Chaki, Hisaaki**
  **Kaminiikawa-gun, Toyama-ken (JP)**
- **Maekawa, Mutsuko**
  **Toyama-shi (JP)**
- **Todo, Yozo**
  **Toyama-shi (JP)**
- **Narita, Hirokazu**
  **Toyama-shi (JP)**

(74) Representative:
**Wächtershäuser, Günter, Prof. Dr.**
**Patentanwalt,**
**Tal 29**
**80331 München (DE)**

(56) References cited:
**WO-A-88/08424**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 589 484 B1

**Description**

This invention relates to a 1,2-ethanediol derivative and a salt thereof, a process for producing the same, and a cerebral function-improving agent comprising the same. The cerebral function-improving agent of this invention is useful for treating cerebrovascular dementia, senile dementia, Alzheimer's dementia, sequelae of ischemic encephalopathy and cerebral apoplexy.

As 1,2-ethanediol derivatives, there are known, for example, those described in U.S. Patent No. 2,928,845; J. Pharm. Sci., vol. 50, pp. 769-771 (1961); Farmaco. Ed. Sci., vol. 19, pp. 1056-1065 (1964); etc.

These compounds are in use as a local anesthetic. However, nothing is known as to their use as a cerebral function-improving agent, an antiamnesic agent or a nootropic agent.

WO No. 88/8424 describes that 1,2-ethanediol derivatives can be used for the remedy of Alzheimer's disease and other degenerative neurological disorders. However, no specific description or example of these derivatives is given in the application.

Drugs such as cerebral metabolic enhancers, cerebrovasodilators and the like are currently in use for the remedy of various dementias, particularly dementia of Alzheimer's type and cerebrovascular dementia.

However, no cerebral function-improving agent has been found as yet which is useful for treating cerebrovascular dementia, senile dementia, Alzheimer's dementia, sequelae of ischemic encephalopathy and cerebral apoplexy.

An object of this invention is to provide a novel 1,2-ethanediol derivative and a salt thereof.

Another object of this invention is to provide a process for producing a novel 1,2-ethanediol derivative and a salt thereof.

Still another object of this invention is to provide a novel cerebral function-improving agent which is useful for treating cerebrovascular dementia, senile dementia, Alzheimer's dementia, sequelae of ischemic encephalopathy and cerebral apoplexy and yet has little side effect.

The present inventors have made study in order to solve the above-mentioned problems. As a result, it has been found that a 1,2-ethanediol derivative represented by the following general formula [I] or a salt thereof has an excellent antiamnesic activity and an excellent antihypoxic activity and is very useful as a cerebral function-improving agent:

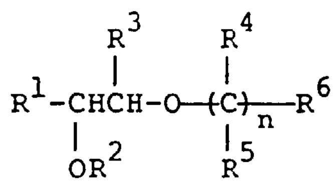

[I]

wherein $R^1$ represents a substituted or unsubstituted naphthyl, indanyl, indenyl or tetrahydronaphthyl group; $R^2$ represents a hydrogen atom, a lower alkyl group or a hydroxyl-protecting group; $R^3$ represents a hydrogen atom or a lower alkyl group; $nR^4$'s and $nR^5$'s are the same as or different from one another and represent hydrogen atoms or lower alkyl groups; $R^6$ represents an ammonio group or a substituted or unsubstituted amino or nitrogen-containing heterocyclic group, the nitrogen-containing heterocyclic group being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6; wherein the substituent on $R^1$ is selected from the group consisting of halogen atoms, substituted or unsubstituted amino, lower alkyl, aryl, ar-lower alkyl, lower alkoxy, ar-lower alkoxy, aryloxy, carbamoyloxy, lower alkylthio, lower alkenyl, lower alkenyloxy, ar-lower alkylthio, ar-lower alkylsulfonyl, arylsulfonyl, lower alkylsulfonylamino, arylsulfonylamino and heterocyclic groups and protected amino groups, protected or unprotected hydroxyl groups, nitro group, oxo group and lower alkylenedioxy groups; the substituted lower alkyl, aryl, ar-lower alkyl, lower alkoxy, ar-lower alkoxy, aryloxy, carbamoyloxy, lower alkylthio, lower alkenyl, lower alkenyloxy, ar-lower alkylthio, ar-lower alkylsulfonyl, arylsulfonyl, lower alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted lower alkyl groups, lower alkyl groups substituted by a protected or unprotected hydroxyl group, unsubstituted or halogen-substituted aryl groups, unsubstituted or halogen-substituted aroyl groups, unsubstituted lower alkoxy groups, lower alkoxy groups substituted by a lower alkoxy group, lower acyl groups, ar-lower alkyl groups, ar-lower alkenyl groups, heterocyclic groups, heterocyclic-CO- groups, oxo group, lower alkylsulfonyl groups and arylsulfonyl groups; and the substituted amino group as the substituent of $R^1$ and the substituted amino group as $R^6$ have each at least one substituent selected from the group consisting of protected or unprotected

hydroxyl groups, unsubstituted lower alkyl groups, lower alkyl groups substituted by a protected or unprotected carboxyl or hydroxyl group, cycloalkyl groups, aryl groups, lower acyl groups, ar-lower alkyl groups, heterocyclic groups, unsubstituted or oxo-substituted heterocyclic-CO- groups, adamantyl group, lower alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo-[2,3-a]pyridyl, benzo[b]piperazinyl, chromenyl, isothiazolyl, isoxazolyl, oxadiazolyl, pyridazinyl, isoindolyl and isoquinolyl groups.

Incidentally, the cerebral function-improving agent mentioned herein refers to a cerebral function-improving agent having not only effects possessed by conventional cerebral function-improving agents, for example, sequelae of ischemic encephalopathy and cerebral apoplexy but also therapeutic or prophylactic effects for amnesia and dementias (e.g. cerebrovascular dementia, senile dementia and Alzheimer's dementia).

In the present specification, the following terms have the following definitions unless otherwise specified.

The term "halogen atom" means, for example, a fluorine atom, a chlorine atom, a bromine atom and an iodine atom; the term "lower alkyl group" means $C_{1-6}$ alkyl groups such as methyl, ethyl, n-propyl, isopropyl, n-butyl, isobutyl, tert-butyl, pentyl, hexyl and the like; the term "lower alkoxy group" means $C_{1-6}$ alkyl-O- groups; the term "lower alkylthio group" means $C_{1-6}$ alkyl-S-groups; the term "lower alkenyl group" means $C_{2-6}$alkenyl groups such as vinyl, propenyl, butenyl, pentenyl, hexenyl and the like; the term "lower alkenyloxy group" means $C_{2-6}$alkenyl-O- groups; the term "cycloalkyl group" means $C_{3-6}$cycloalkyl groups such as cyclopropyl, cyclobutyl, cyclopentyl, cyclohexyl and the like; the term "aryl group" means phenyl, naphthyl, indanyl and indenyl groups; the term "aryloxy group" means aryl-O- groups; the term "ar-lower alkyl group" means ar-$C_{1-4}$ alkyl groups such as benzyl, diphenylmethyl, trityl, phenethyl and the like; the term "ar-lower alkoxy group" means ar-$C_{1-4}$alkyl-O- groups; the term "ar-lower alkylthio group" means aryl-$C_{1-4}$alkyl-S- groups; the term "lower alkylenedioxy group" means $C_{1-4}$alkylenedioxy groups such as methylenedioxy, ethylenedioxy and the like; the term "lower acyl group" means $C_{1-6}$acyl groups such as formyl, acetyl, butyryl and the like; the term "aroyl group" means aryl-CO- groups; the term "lower alkylsulfonyl group" means $C_{1-6}$alkyl-$SO_2$- groups; the term "arylsulfonyl group" means aryl-$SO_2$- groups; ther term "ar-lower alkylsulfonyl group" means aryl-$C_{1-6}$alkyl-$SO_2$- groups; the term "lower alkylsulfonyloxy group" means $C_{1-6}$alkyl-$SO_2$-O- groups; the term "arylsulfonyloxy group" means aryl-$SO_2$-O- groups; the term "lower alkylsulfonylamino group" means $C_{1-6}$alkyl-$SO_2$-NH-groups; the term "arylsulfonylamino group" means aryl-$SO_2$NH- groups; the term "di-lower alkylamino group" means di-$C_{1-6}$alkyl-NH- groups and the term "ammonio group" means tri-lower alkylammonio groups such as trimethylammonio, triethylammonio; the term "lower alkoxycarbonyl group" means $C_{1-6}$alkyl-O-CO- groups and the like.

The protective groups for hydroxyl group, carboxyl group and amino group include those conventional protective groups for hydroxyl group, carboxyl group and amino group which are described in Protective Groups in Organic Synthesis [Theodra W. Greene (1981) John Wiley & Sons, Inc.]. In particular, the protective group for hydroxyl group specifically includes, for example, lower alkyl, lower acyl, tetrahydropyranyl and ar-lower alkyl groups such as substituted or unsubstituted benzyl.

The salt of the 1,2-ethanediol derivative represented by the general formula [I] can be any pharmaceutically acceptable salt. It includes, for example, salts with mineral acids such as hydrochloric acid, hydrobromic acid, sulfuric acid, phosphoric acid and the like; salts with carboxylic acids such as formic acid, acetic acid, oxalic acid, fumaric acid, maleic acid, malic acid, tartaric acid, aspartic acid and the like; salts with sulfonic acids such as methanesulfonic acid, benzenesulfonic acid, p-toluenesulfonic acid, naphthalenesulfonic acid and the like; and salts with alkali metals such as sodium, potassium and the like.

When the 1,2-ethanediol derivative of the general formula [I] or its salt has isomers (e.g. optical isomers, geometrical isomers, tautomers), all of these isomers are included in this invention. Also, the hydrate, solvate and all crystal forms of the compound of this invention are included in this invention.

Next, there is described a process for producing a 1,2-ethanediol derivative of the general formula [I] or a salt thereof.

The 1,2-ethanediol derivative of the general formula [I] or its salt can be produced by per se known processes or their appropriate combinations, for example, the following production processes.

Production Process 1

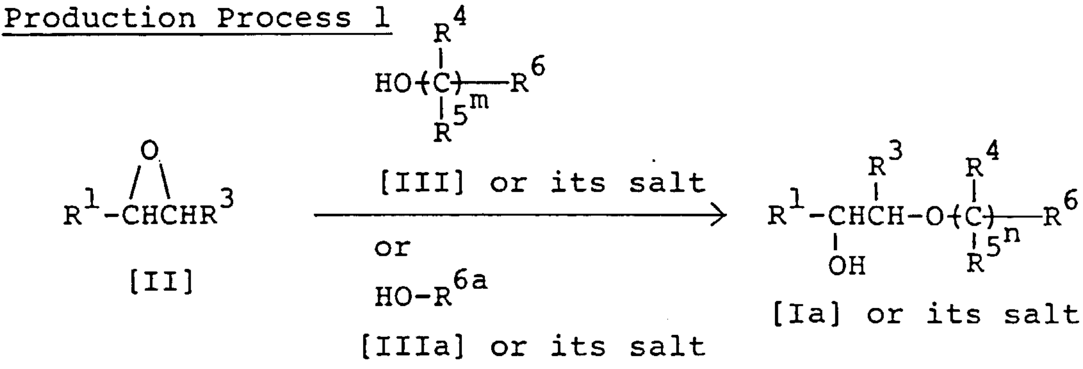


[II]

[Ia] or its salt

Production Process 2

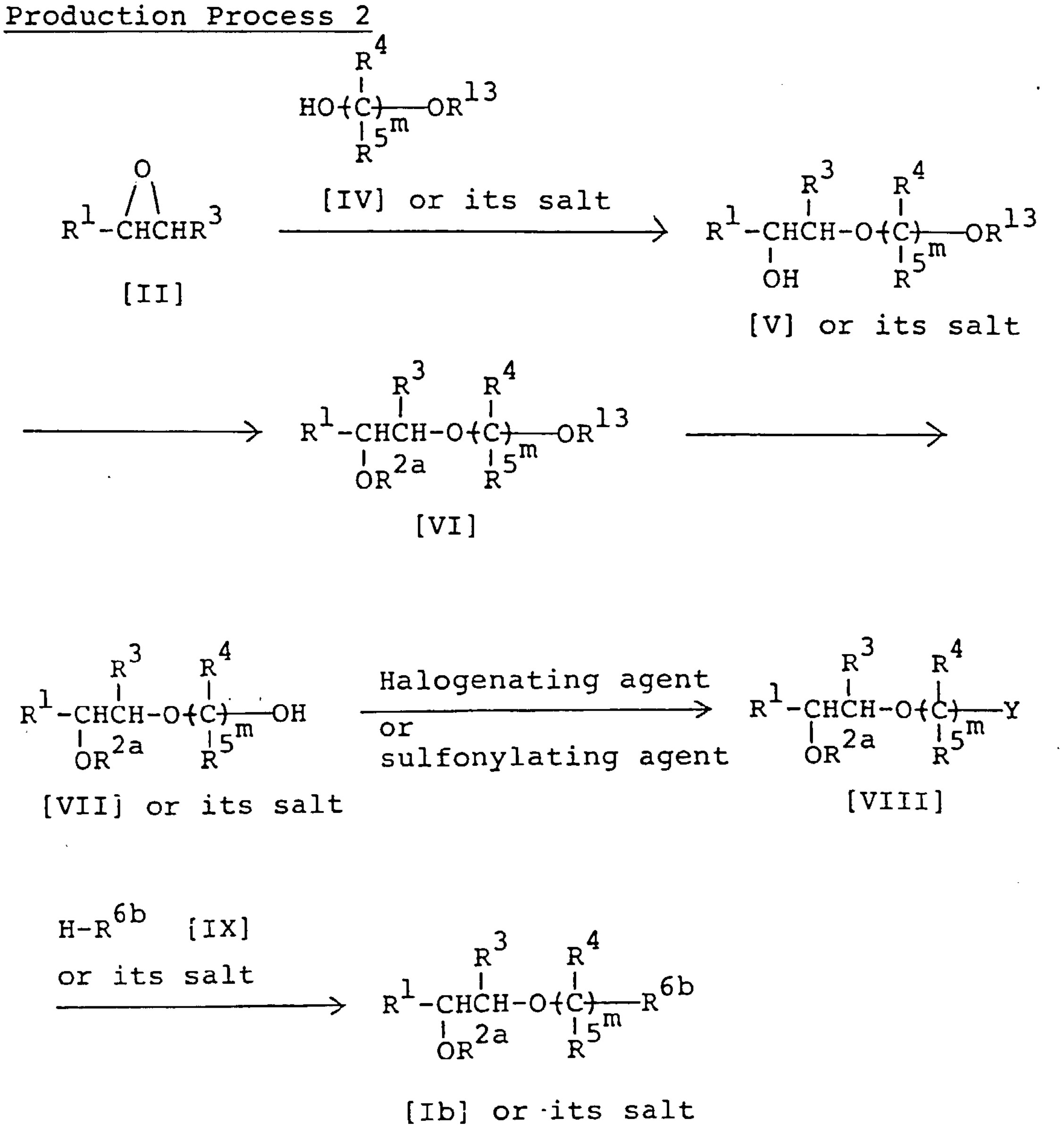


[II]

[V] or its salt

[VI]

[VII] or its salt

[VIII]

[Ib] or its salt

Production Process 3

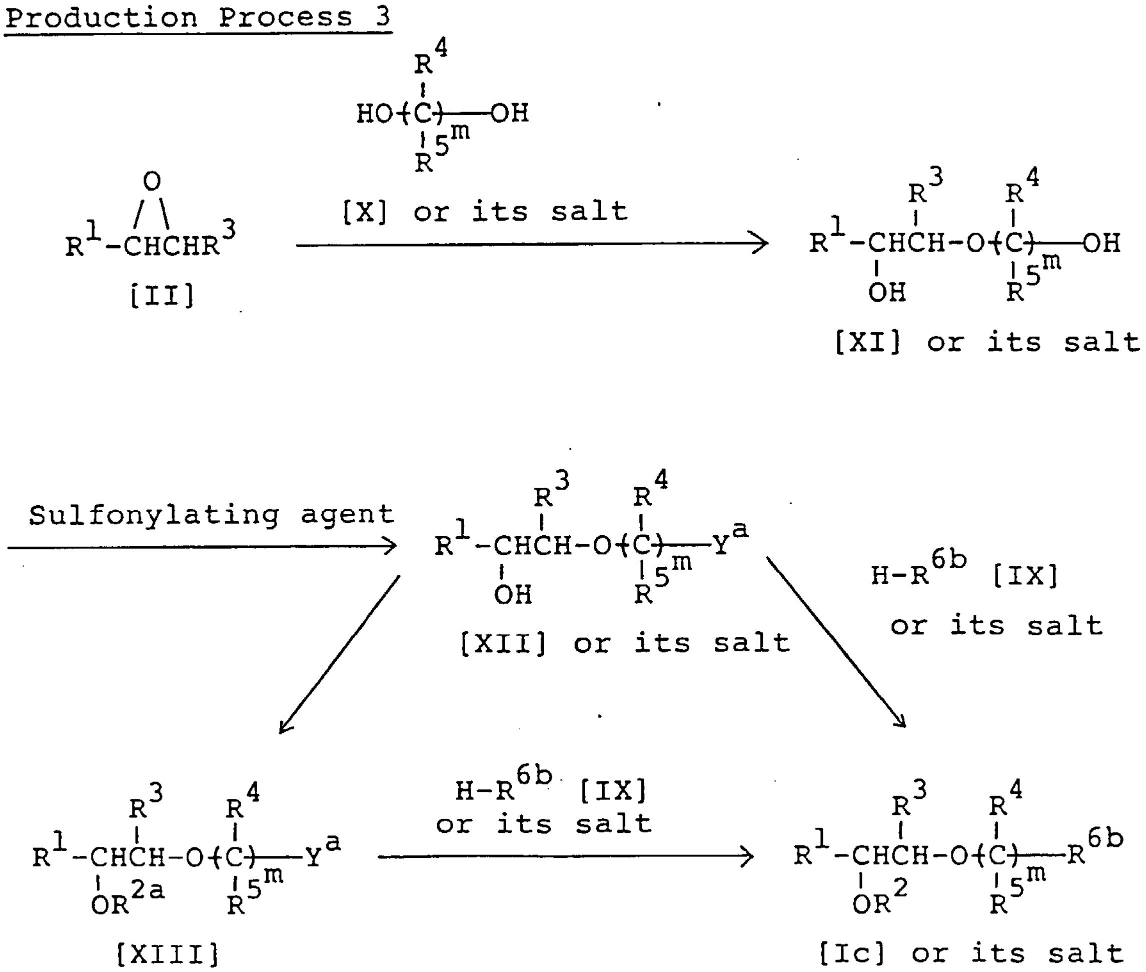


Production Process 4

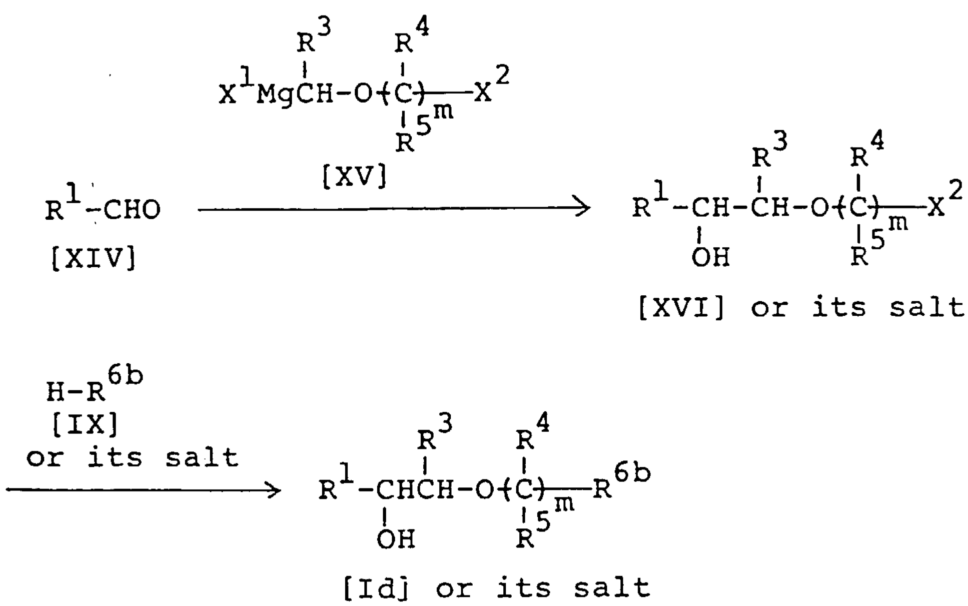

In the above reaction schemes, $R^1$, $R^2$, $R^3$, $R^4$, $R^5$, $R^6$ and n have the same meanings as defined above; $R^{2a}$ represents the same hydroxyl-protecting group as in the definition of $R^2$; $R^{6a}$ represents the same substituted or unsubstituted nitrogen-containing heterocyclic group as in the definition of $R^6$, provided that the heterocyclic group has a free valence on a carbon atom forming the heterocyclic ring; $R^{6b}$ represents the same substituted or unsubstituted nitrogen-containing heterocyclic group as in the definition of $R^6$, provided that the nitrogen-containing heterocyclic group has a free valence on a nitrogen atom forming the heterocyclic ring, or a substituted or unsubstituted amino group; $R^{13}$ represents the same hydroxyl-protecting group as in the definition of $R^2$; $X^1$ and $X^2$, which may be the same or different, represent halogen atoms, Y represents a removable group such as a halogen atom, a lower alkylsulfonyloxy group, an arylsulfonyloxy group or the like; $Y^a$ represents an arylsulfonyloxy group; and m represents an integer of 1-6.

As the salts of the compounds of the general formulas [III], [IIIa], [IV], [V], [VII], [IX], [X], [XI], [XII], [XVI], [Ia], [Ib], [Ic] and [Id], there can be mentioned the same salts as the salts of the compound of the general formula [I].

Next, each of the above production processes is described.

Production Process 1

A compound of the general formula [II] is reacted with a compound of the general formula [III] or its salt or a compound of the general formula [IIIa] or its salt in the presence or absence of a base to obtain a compound of the general formula [Ia] or its salt.

The solvent to be used in this reaction can be any solvent unless it adversely affects the reaction. There can be mentioned, for example, aromatic hydrocarbons such as benzene, toluene, xylene and the like; sulfoxides such as dimethylsulfoxide and the like; amides such as N,N-dimethylformamide and the like; and ethers such as tetrahydrofuran, dioxane and the like. These solvents can be used alone or in admixture of two or more. It is also possible to use the compound of the general formula [III] or [IIIa] as the solvent.

As the base to be used optionally, there can be mentioned, for example, sodium hydride, metallic sodium and potassium tert-butoxide.

In the reaction, the compound of the general formula [III] or its salt, or the compound of the general formula [IIIa] or its salt is used in an amount of 1-100 moles, preferably 1-10 moles, per mole of the compound of the general formula [II].

The base as an optional component is used in an amount of 0.01-1.2 moles per mole of the compound of the general formula [II].

This reaction can be effected usually at 20-150°C, preferably 70-90°C, for 1 minutes to 24 hours, preferably 5 minutes to 5 hours.

Production Process 2

(1) A compound of the general formula [II] is reacted with a compound of the general formula [IV] or its salt in the presence or absence of a base to obtain a compound of the general formula [V] or its salt.

This reaction can be effected in the same manner as described in Production Process 1.

The obtained compound of the general formula [V] or its salt may be used in the subsequent reaction without being isolated.

(2) The compound of the general formula [V] or its salt is subjected to conventional reaction for protection of hydroxyl group to obtain a compound of the general formula [VI].

The obtained compound of the general formula [VI] may be used in the subsequent reaction without being isolated.

Then, the compound of the general formula [VI] is subjected to reaction for selective removal of the hydroxyl-protecting group to obtain a compound of the general formula [VII] or its salt.

The obtained compound of the general formula [VII] or its salt may be used in the subsequent reaction without being isolated.

These reactions can be effected according to per se known methods, for example, the method described in Protective Groups in Organic Synthesis [Theodra W. Green (1981), John Wiley & Sons, Inc.] or a method similar thereto.

The combination of the hydroxyl-protecting groups ($R^{13}$ and $R^{2a}$) to be used in these reactions can be selected appropriately.

(3) The compound of the general formula [VII] or its salt is reacted with a halogenating agent or a sulfonylating agent in a solvent in the presence or absence of a base to obtain a compound of the general formula [VIII].

The solvent to be used in the reaction can be any solvent unless it adversely affects the reaction. There can be mentioned, for example, halogenated hydrocarbons such as methylene chloride, chloroform and the like; ethers such as tetrahydrofuran, dioxane and the like; nitriles such as acetonitrile and the like; and amides such as N,N-dimethylformamide and the like. These solvents can be used alone or in admixture of two or more.

As the base to be used optionally, there can be mentioned, for example, organic and inorganic bases such as

triethylamine, diisopropylethylamine, 1,8-diazabicyclo-[5.4.0]undec-7-ene (DBU), pyridine, potassium tert-butoxide, sodium carbonate, potassium carbonate, sodium hydride and the like.

As the halogenating agent, there can be mentioned, for example, phosphorus oxychloride, phosphorus oxybromide, phosphorus trichloride, phosphorus pentachloride, thionyl chloride and the like.

As the sulfonylating agent, there can be mentioned, for example, methanesulfonyl chloride, p-toluenesulfonyl chloride and the like.

Each of the halogenating agent, the sulfonylating agent and the base as an optional component is used in an amount of at least 1 mole, preferably 1-2 moles, per mole of the compound of the general formula [VII] or its salt.

This reaction can be effected usually at -10° to 100°C, preferably 0° to 40°C, for 10 minutes to 30 hours.

The obtained compound of the general formula [VIII] may be used as it is, in the subsequent reaction without being isolated.

(4) The compound of the general formula [VIII] is reacted with a compound of the general formula [IX] or its salt in the presence or absence of a catalyst in the presence or absence of a base to obtain a compound of the general formula [Ib] or its salt.

The solvent to be used in this reaction can be any solvent unless it adversely affects the reaction. There can be mentioned, for example, the same solvents as mentioned in (3) of Production Process 2.

As the catalyst to be used optionally, there can be mentioned, for example, potassium iodide, sodium iodide and the like.

The catalyst is used in an amount of 0.1-1 mole per mole of the compound of the general formula [VIII].

As the base to be used optionally, there can be mentioned, for example, the same bases as mentioned in (3) of Production Process 2.

Each of the compound of the general formula [IX] or its salt and the base as an optional component is used in an amount of at least 1 mole, preferably 1-20 moles, per mole of the compound of the general formula [VIII].

This reaction can be effected usually at 10-150°C, preferably 20-100°C for 10 minutes to 20 hours.

Production Process 3

(1) A compound of the general formula [II] is reacted with a compound of the general formula [X] or its salt in the presence or absence of a base to obtain a compound of the general formula [XI] or its salt.

This reaction can be effected in accordance with the same method as mentioned in Production Process 1.

(2) The compound of the general formula [XI] or its salt is reacted with a sulfonylating agent in a solvent in the presence or absence of a base to obtain a compound of the general formula [XII] or its salt.

The solvent to be used in this reaction can be any solvent unless it adversely affects the reaction. There can be mentioned, for example, the same solvents as mentioned in (3) of Production Process 2.

As the base to be used optionally, there can be mentioned, for example, the same bases as mentioned in (3) of Production Process 2.

As the sulfonylating agent, there can be mentioned, for example, p-toluenesulfonyl chloride and the like.

Each of the sulfonylating agent and the base as an optional component is used in an amount of at least 0.95 mole, preferably 1-2 moles, per mole of the compound of the general formula [XI] or its salt.

This reaction can be effected usually at -10° to 100°C, preferably 0° to 40°C, for 10 minutes to 30 hours.

The obtained compound of the general formula [XII] or its salt may be used in the subsequent reaction without being isolated.

(3) The compound of the general formula [XII] or its salt is subjected to conventional reaction for protection of hydroxyl group to obtain a compound of the general formula [XIII].

The reaction can be effected in accordance with per se known methods, for example, the method described in Protective Groups in Organic Synthesis [Theodra W. Green (1981), John Wiley & Sons, Inc.] or a method similar thereto.

The obtained compound of the general formula [XIII] may be used in the subsequent reaction without being isolated.

(4) The compound of the general formula [XII] or its salt or the compound of the general formula [XIII] is reacted with compound of the general formula [IX] or its salt in the presence or absence of a base to obtain a compound of the general formula [Ic] or its salt.

This reaction can be effected in the same method as described in (4) of Production Process 2.

Production Process 4

(1) A compound of the general formula [XIV] is reacted with a compound of the general formula [XV] to obtain a compound of the general formula [XVI] or its salt.

The solvent to be used in this reaction can be any solvent unless it adversely affects the reaction. There can be mentioned, for example, ethers such as diethyl ether, tetrahydrofuran, dioxane and the like; and aromatic hydrocarbons

such as benzene, toluene and the like. These solvents can be used alone or in admixture of two or more.

In this reaction, the compound of the general formula [XV] is used in an amount of 0.8-100 moles, preferably 0.8-10 moles, per mole of the compound of the general formula [XIV].

This reaction can be effected usually at -78° to 100°C, preferably -78° to 50°C for 5 minutes to 24 hours.

The obtained compound of the general formula [XVI] or its salt may be used in the subsequent reaction without being isolated.

Incidentally, the compound of the general formula [XV] to be used in this reaction can be produced according to a per se known method, for example, the method described in Bull. Soc. Chim. Fr., 1967 (5), pp. 1533-40.

(2) The compound of the general formula [XVI] or its salt is reacted with a compound of the general formula [IX] or its salt in the presence or absence of a base to obtain a compound of the general formula [Id] or its salt.

The solvent to be used in this reaction can be any solvent unless it adversely affects the reaction. There can be mentioned, for example, halogenated hydrocarbons such as methylene chloride, chloroform and the like; ethers such as tetrahydrofuran, dioxane and the like; alcohols such as ethanol, propanol, butanol and the like; nitriles such as acetonitrile and the like; and amides such as N,N-dimethylformamide and the like. These solvents can be used alone or in admixture of two or more.

As the base to be used optionally, there can be mentioned, for example, the same bases as mentioned in (3) of Production Process 2.

Each of the compound of the general formula [IX] or its salt and the base as an optional component is used in an amount of at least 1 mole, preferably 1-20 moles, per mole of the compound of the general formula [XVI] or its salt.

This reaction can be effected usually at 10-150°C, preferably 20-100°C, for 10 minutes to 20 hours.

In the above production processes, the reactants or base can also be used as solvent depending on the nature of the reactants or base.

In the above production processes, when the compounds of the general formulas [II], [III], [IIIa], [IV], [V], [VI], [VII], [VIII], [IX], [X], [XI], [XII], [XIII], [XIV], [XV] and [XVI], have isomers (e.g. optical isomers, geometrical isomers, tautomers), the compounds can be used in any isomer form. Further, the compounds can be used in a hydrate form, a solvate form or any crystal form.

When the compounds of the general formulas [II], [III], [IIIa], [IV], [V], [VI], [VII], [VIII], [IX], [XII], [XIII], [XIV], [XV], [XVI], [I], [Ia], [Ib], [Ic] and [Id] have a hydroxyl group, an amino group or a carboxyl group, it is possible to previously protect these groups with a conventional protective group and to remove, after the reaction, the protective group, if necessary, according to a per se known method.

The compound thus obtained may be subjected to conventional isolation and purification procedures such as column chromatography, crystallization, distillation, extraction and the like.

The 1,2-ethanediol derivative of the general formula [I] or its salt can be converted into other 1,2-ethanediol derivative of the general formula [I] or its salt by subjecting the former compound to appropriate combination of per se known reactions such as oxidation reaction, reduction reaction, addition reaction, acylation reaction, alkylation reaction, sulfonylation reaction, deacylation reaction, substitution reaction, dehydration reaction, hydrolysis reaction and the like.

The compound of the general formula [II] which is the starting material for producing the compound of this invention can be produced by per se known processes, for example, the process described in JACS, vol. 87, p.1353 (1965) and the process described in Shin Jikken Kagaku Koza, vol. 14, p. 579 (1977), Maruzen.

The compound of this invention, when used as a drug, may be appropriately mixed with excipients such as filler, carrier, diluent and the like and can be formed into tablets, capsules, powders, granules, fine granules, pills, suspensions, emulsions, liquids, syrups, injections, etc. according to conventional methods. These drugs can be administered orally or parenterally. The dosage route, dose and number of administrations can be appropriately varied depending upon the age, weight and symptom of patient, but in the case of oral administration, generally 0.01-500 mg of the present compound can be administered daily to an adult patient in one to several portions.

Next, the pharmacological activities of representative compounds of this invention are described.

The numbers of the test compounds used in the following pharmacological tests refer to the numbers of the compounds shown in Production Examples appearing hereinafter.

1. Effect of test compound on hypoxia

A test compound dissolved in physiological saline (100 mg/kg) was orally administered to a ddY female mouse (5-6 weeks old, each group consisting of 10 mice). After 1 (or 30 minutes*) hour from the administration, each mouse was placed in a 300-ml glass chamber, and a gas mixture consisting of 4% of oxygen and 96% of nitrogen was passed through the chamber at a rate of 5 liters/min. A time from the start of gas passing to the death of each mouse was measured.

To a control mice group was orally administered only physiological saline.

The antihypoxic activity of the test compound was calculated from the following formula:

$$\frac{\text{Mean survival time of mice of administration group}}{\text{Mean survival time of mice of control group}} \times 100\ (\%)$$

The results are shown in Table 1.

Table 1

| Compound No. | Antihypoxic activity (%) |
|---|---|
| 1 | 144 |
| 2 | 123 |
| 3 | 177 |
| 4 | 246 |
| 5 | 150 |
| 9 | 177 |
| 10 | 149 |
| 11 | 123 |
| 12 | 150 |
| 16 | 159 |
| 19 | 128 |
| 20 | 124 |
| 23 | 145* |
| 24 | 186* |
| 25 | 190* |
| 29 | 154 |
| 32 | 155* |
| 33 | 124 |
| 34 | 126 |
| 44 | 150* |
| 45 | 185* |
| 52 | 198 |
| 55 | 160* |
| 56 | 183 |
| 62 | 157* |
| 75 | 182* |
| 76 | 168 |
| 82 | 198* |

Note: * Mice were placed in the chamber after 30 minutes from the administration instead of after 1 hr from the administration.

2. Effect of test compound on amnesia

(1) Electroconvulsive shock (ECS)-induced amnesia

A test compound dissolved in physiological saline was intraperitoneally administered to a ddY male mouse (5-6 weeks old, each group consisting of 10 mice). The acquisition trial in passive avoidance task was carried out after 1 hour from the administration. Each mouse was placed in the bright compartment of a two-compartment step-through type passive avoidance apparatus consisting of a bright compartment and a dark compartment (MPA-100M manufactured by Muromachi Kikai). When the mouse entered the dark compartment, the guilotine door of the dark compartment was closed; after 0.5 second, an inescapable footshock (1.6 mA, 3 seconds) was delivered. Immediately thereafter, ECS (25 mA, 0.5 second) was applied through the both eyes of the mouse. After 24 hours, in the retention trial, the mouse was again placed in the bright compartment and the response latency for mouse to enter the dark compartment was measured. If the mouse avoided longer than 300 seconds, a ceiling score of 300 seconds was assigned.

A control mice group to which only physiological saline had been administered intraperitoneally, and response latency was also measured in the same manner.

Antiamnesic activity was taken as a median of the response latencies of the 10 mice and expressed by the following symbols:

- - : 0-60 seconds
- + : 61-100 seconds
- ++ : 101-150 seconds
- +++ : 151-300 seconds

The results are shown in Table 2.

Table 2

| Compound No. | Dosage (mg/Kg) | Antiamnesic activity |
|---|---|---|
| 1 | 10 | + |
| 2 | 3 | + |
| 8 | 10 | ++ |
| 10 | 3 | ++ |
| 12 | 10 | + |
| 16 | 3 | ++ |
| 25 | 3 | +++ |
| 34 | 3 | + |
| 39 | 3 | ++ |

(2) Effect of test compound on cycloheximide-induced amnesia

It was reported by Yamazaki et al. [Drugs, Mind and Action, vol. 3, pp. 127-136 (1983)] that cycloheximide interfers with the retrieval process of memory of mouse. Hence, the following test was carried out.

A test was carried out in accordance with the method described in Drugs, Mind and Action, vol. 3, pp. 127-136 (1983) and Folia Pharmacologica Japonica, vol. 89, pp. 243-252 (1987).

As the test apparatus, there was used a step-down type passive avoidance training box. It was a black acrylic resin box of 22 cm x 22 cm x 21 cm (height) whose floor portion consisted of a stainless steel grid and which had, at one corner of the floor grid, a platform of 7 cm x 7 cm x 2 cm (height).

Cycloheximide was dissolved in physiological saline, and injected subcutaneously at 120 mg/kg to a ddY male mouse (5-6 weeks old, each group consisting of 10 mice). In an acquisition trial, after 15 minutes from the administration, each mouse was placed on the platform in the above test apparatus. As soon as the mouse stepped down the platform, a 2 mA current was delivered for 2 seconds, immediately after which the mouse was returned to its home cage. A retention test was performed 24 hours after the acquisition. To each mouse which had been treated with cycloheximide was orally administered a test compound dissolved in physiological saline; after 30 minutes from the administration, the mouse was again placed on the platform and the response latency for mouse to step down was measured. If the mouse avoided longer than 300 seconds, a ceiling score of 300 seconds was assigned.

A control mice group to which only physiological saline had been administered orally, and response latency was also measured in the same manner.

Antiamnesic activity was taken as a median of the response latencies of the 10 mice and expressed by the following symbols:

- - : 0-60 seconds
- + : 61-100 seconds
- ++ : 101-150 seconds
- +++ : 151-300 seconds

The results are shown in Table 3.

Table 3

| Compound No. | Dosage (mg/Kg) | Antiamnesic activity |
|---|---|---|
| 1 | 3 | ++ |
| 2 | 10 | ++ |
| 3 | 3 | + |
| 5 | 10 | ++ |

Table 3 (continued)

| Compound No. | Dosage (mg/Kg) | Antiamnesic activity |
|---|---|---|
| 6 | 3 | + |
| 7 | 3 | + |
| 8 | 3 | +++ |
| 11 | 3 | + |
| 13 | 3 | + |
| 14 | 3 | ++ |
| 15 | 3 | ++ |
| 17 | 3 | ++ |
| 18 | 10 | ++ |
| 19 | 3 | ++ |
| 21 | 3 | ++ |
| 24 | 3 | +++ |
| 25 | 30 | +++ |
| 30 | 3 | + |
| 33 | 3 | + |
| 34 | 3 | +++ |
| 35 | 3 | + |
| 36 | 10 | + |
| 39 | 30 | + |
| 53 | 10 | + |
| Control | - | - |

3. Inhibitory activity for acetylcholinesterase

A test was conducted in accordance with the method of Ellman et al. [Biochem. Pharmacol., vol. 7, pp. 88-95, 1961].

That is, acetylthiocholine (as a substrate) was added to a phosphate buffer solution containing 5,5'-dithiobis-(2-nitrobenzoic acid) (DTNB), a test compound and a mouse cerebral homogenate (as an acetylcholinesterase source). The resulting mixture was incubated, and the amount of the resulting 5-thio-2-thio-2-nitrobenzoic acid was photometrically measured at 412 nm.

The inhibitory activity for acetylcholinesterase of the test compound was expressed by the inhibition (%) when the final concentration of the test compound was 10 µg/ml.

The results are shown in Table 4.

Table 4

| Compound No. | Inhibition (%) |
|---|---|
| 1 | 30 |
| 3 | 25 |
| 6 | 59 |
| 10 | 44 |
| 12 | 22 |
| 14 | 31 |
| 19 | 30 |
| 20 | 26 |
| 33 | 43 |
| 75 | 38 |

4. Acute toxicity

A test compound dissolved in physiological saline was intravenously administered to a group of 3 ddY male mice (5-6 weeks old) to examine the acute toxicity of the test compound.

As a result, test compound Nos. 229, 235, 236, 240, 251, 256, 279, 283 and 309 gave no death case at 50 mg/kg.

It is easily appreciated from the above test results that the compound of this invention is excellent in antihypoxic activity, antiamnesic activity and inhibitory activity for acetylcholinesterase and has low toxicity.

From the above result, it is also easily appreciated that the cerebral function-improving agent of this invention is useful for treating cerebrovascular dementia, senile dementia, Alzheimer's dementia, sequelae of ischemic encephalopathy and cerebral apoplexy.

Next, the process for producing the compound of this invention is specifically described by way of Production Examples.

In the Production Examples, the mixing ratio of eluant is by volume in all cases and, as the carrier in column chromatography, there was used a silica gel (Kieselgel 60, Art. 7734) manufactured by Merck Co.

The abbreviations used in the Production Examples have the following meanings.

Me: methyl, Et: ethyl, i-Pr: isopropyl, t-Bu: tert-butyl, Ac: acetyl, Bz: benzyl, IPA: isopropyl alcohol, IPE: diisopropyl ether.

In the following sentences and tables, the substances in [ ] refer to solvents used in recrystallization.

Production Example 1

A mixture of 13.2 g of potassium tert-butoxide and 47.2 ml of 2-(N,N-dimethylamino)ethanol was heated to 80°C. Thereto was dropwise added 40.0 g of 2-(1-naphthyl)oxirane in 3.5 hours. The resulting mixture was stirred for 1.5 hours at 80-85°C. The reaction mixture was cooled and added to a mixture of 100 ml of ethyl acetate and 100 ml of ice water. The resulting mixture was adjusted to pH 11.5 with 6 N hydrochloric acid. The organic layer was separated. The aqueous layer was extracted with 50 ml of ethyl acetate. The extract was combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was subjected to distillation to obtain a fraction having a boiling point of 152-163°C/ 0.6-0.8 mmHg. The oily product obtained was dissolved in 200 ml of acetone. Into the solution was blown hydrogen chloride gas with ice cooling. The resulting crystals were collected by filtraiton, washed with acetone, and dried to obtain 22.7 g of 2-[2-(N,N-dimethylamino)ethoxy]-1-(1-naphthyl)ethanol hydrochloride (compound No. 1) Melting point: 196-197°C [EtOH]

The compounds shown in Table 5 were obtained in the same manner.

In Table 5, $R^1$, $R^2$, $R^3$, $R^{4a}$, $R^{4b}$, $R^6$, na and nb each show a substituent or integer used in the following formula:

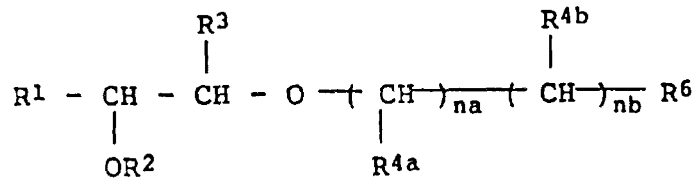

Table 5

| Compound No. | R1 | R2 | R3 | R4a | R4b | R6 | na | nb | Addition salt | Melting point (°C) |
|---|---|---|---|---|---|---|---|---|---|---|
| 2 | 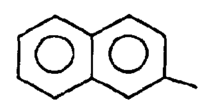 | H | H | H | H | 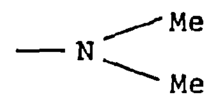 | 1 | 1 | HCl | 193-194 [EtOH] |
| 3 | 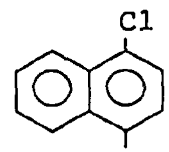 | " | " | " | " | " | " | " | " | 149-150 [$Me_2CO$-EtOH] |
| 4 | 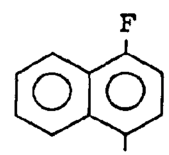 | " | " | " | " | " | " | " | " | 178-179 [$Me_2CO$-EtOH] |
| 5 | 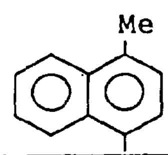 | " | " | " | " | " | " | " | " | 184-185 [$Me_2CO$-EtOH] |

- Cont'd -

Table 5 (Cont'd)

| | | | | | | | | | | |
|---|---|---|---|---|---|---|---|---|---|---|
| 6* | 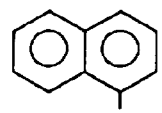 | H | H | H | H | 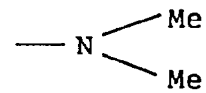 | 2 | 1 | - | Oily |
| 7* | " | " | " | " | " | " | " | 2 | " | " |
| 8 | 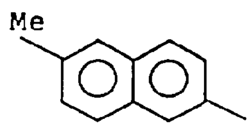 | " | " | " | " | " | 1 | 1 | HCl | 213-214 [IPA] |
| 9 | 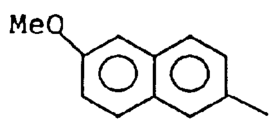 | " | " | " | " | " | " | " | " | 205-205.5 [MeOH-EtOH] |
| 10 | 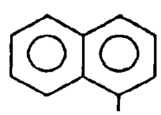 | " | " | " | " | 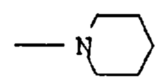 | " | " | " | 174-174.5 [EtOH-$Et_2O$] |

- Cont'd -

Table 5 (Cont'd)

| | | | | | | | | | | |
|---|---|---|---|---|---|---|---|---|---|---|
| 11 | 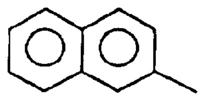 | H | H | H | H | 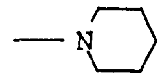 | 1 | 1 | HCl | 156.5-158 [EtOH-$Et_2O$] |
| 12 | 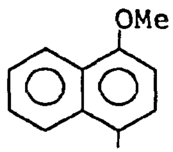 | " | " | " | " | 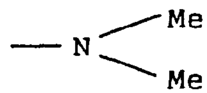 | " | " | " | 157-158 [IPA] |
| 13* | 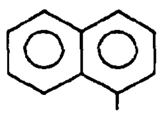 | " | " | " | " | 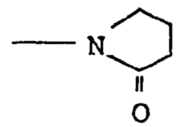 | 1 | 1 | - | Oily |
| 14* | " | " | " | " | " | 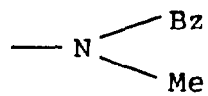 | " | " | " | " |
| 15* | " | " | " | " | " | 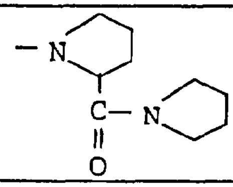 | " | " | " | " |

- Cont'd -

Table 5 (Cont'd)

| | | | | | | | | | | |
|---|---|---|---|---|---|---|---|---|---|---|
| 16 | 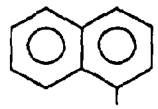 | H | H | H | H | 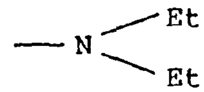 | 1 | 1 | HCl | 155.5-157 [IPA] |
| 17 | 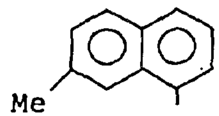 | " | " | " | " | 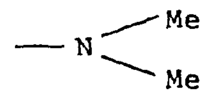 | " | " | " | 151.5 - 153.5 [IPA] |
| 18 | 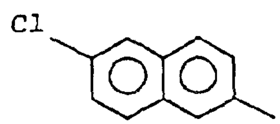 | " | " | " | " | " | " | " | " | 193.5 - 196.5 [EtOH] |
| 19 | 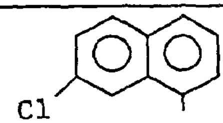 | " | " | " | " | " | " | " | " | 166.5-168 [IPA] |
| 20 | 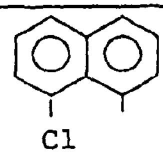 | " | " | " | " | " | " | " | " | 161-163 [EtOH] |

- Cont'd -

Table 5 (Cont'd)

| | | | | | | | | | | |
|---|---|---|---|---|---|---|---|---|---|---|
| 21 | 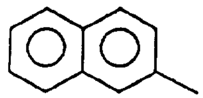 | H | H | H | H | 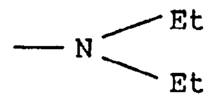 | 1 | 1 | HCl | 100.5 - 101.5 [IPA-AcOEt] |
| 22 | 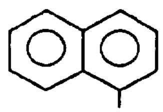 | " | Me | " | " | 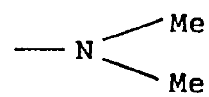 | " | " | " | 186.5-188 [EtOH-$Me_2CO$] |
| 23 | " | " | H | Me | " | " | " | " | Fumaric acid | 197-198.5 [EtOH-AcOEt] |
| 24 | 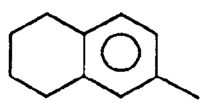 | " | " | H | " | " | " | " | HCl | 199.5-201 [IPA-AcOEt] |
| 25 | 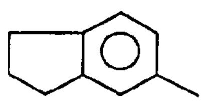 | " | " | " | " | " | " | " | " | 198-199.5 [IPA] |

- Cont'd -

Table 5 (Cont'd)

| | | | | | | | | | | |
|---|---|---|---|---|---|---|---|---|---|---|
| 26 | 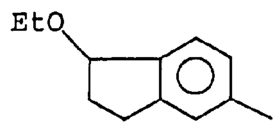 | H | H | H | H | 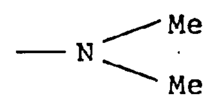 | 1 | 1 | HCl | 157-159 [IPA] |

Note: *: These compounds were not subjected to a step of converting into a hydrochloride.

Production Example 2

(1) A mixture of 6.0 g of 2-hydroxymethyl-4-tritylmorpholine, 1.0 g of potassium tert-butoxide and 6 ml of dimethyl sulfoxide was heated to 80°C. Thereto was dropwise added, at 80-85°C in 2 hours, a solution of 2.8 g of 2-(2-naphthyl) oxirane dissolved in 6 ml of dimethyl sulfoxide. The resulting mixture was stirred at the same temperature for 3 hours. The reaction mixture was cooled and added to a mixture of 60 ml of ice water and 60 ml of ethyl acetate. The organic layer was separated, washed with water and a saturated aqueous sodium chloride solution in this order, and dried over anhydrous magnesium sulfate. The solvent was removed by distillation under reduced pressure to obtain 9.0 g of oily 1-(2-naphthyl)-2-[(4-tritylmorpholin-2-yl)-methoxy] ethanol (compound No. 27).

In the same manner, there was obtained oily 1-(1-naphthyl)-2-[(4-tritylmorpholin-2-yl)methoxy]-ethanol (compound No. 28).

(2) In 50 ml of acetone was dissolved 9.0 g of 1-(2-naphthyl)-2-[(4-tritylmorpholin-2-yl)methoxy]-ethanol. To the solution was added 3.5 ml of a 5.9 N dry hydrogen chloride-ethanol solution with ice cooling. The mixture was stirred for 2 hours at room temperature to effect reaction. After the completion of the reaction, the solvent was removed by distillation under reduced pressure. To the residue thus obtained were added 50 ml of water and 30 ml of ethyl acetate. The aqueous layer was separated and washed with 30 ml of ethyl acetate. Thereto was added 50 ml of ethyl acetate. The resulting mixture was adjusted to pH 10.5 with potassium carbonate. The organic layer was separated, washed with a saturated aqueous sodium chloride solution, and dried over anhydrous magnesium sulfate. The solvent was removed by distillation under reduced pressure. The residue thus obtained was purified by column chromatography (eluant: chloroform/methanol = 5/1) to obtain 1.2 g of an oily product. The oily product was dissolved in 5 ml of isopropanol. To the solution was added 0.5 g of fumaric acid. The mixture was heated to obtain a solution. The solution was allowed to stand at room temperature overnight. The resulting crystals were collected by filtration to obtain 0.9 g of 1-(2-naphthyl)-2-[(morpholin-2-yl)methoxy]ethanol 1/2 fumarate (compound No. 29).

Melting point: 141-144°C [EtOH]

In the same manner, there was obtained amorphous 1-(1-naphthyl)-2-[(mropholin-2-yl)methoxy]ethanol hydrochloride (compound No. 30).

Production Example 3

(1) A mixture of 4.5 g of potassium tert-butoxide and 45 ml of ethylene glycol was heated to 80°C. Thereto was dropwise added 13.7 g of 2-(1-naphthyl)oxirane in 1 hour. The resulting mixture was stirred for 1 hour at the same temperature. The reaction mixture was cooled and added to a mixture of 50 ml of ethyl acetate and 50 ml of ice water. The organic layer was separated. The aqueous layer was extracted twice each with 20 ml of ethyl acetate. The extracts were combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was purified by column chromatography (eluant: toluene/ethyl acetate = 1/3) to obtain 8.3 g of 2-(2-hydroxyethoxy)-1-(1-naphthyl)-ethanol.

Melting point: 91-92°C [IPE]

In the same manner, there was obtained 2-(2-hydroxyethoxy)-1-(2-naphthyl)ethanol.

Melting point: 105-106°C [AcOEt]

(2) 8.3 g of 2-(2-hydroxyethoxy)-1-(1-naphthyl)-ethanol was dissolved in 50 ml of pyridine. The solution was cooled to -25°C. Thereto was added 6.8 g of p-toluenesulfonyl chloride. The resulting mixture was allowed to stand at 0-5°C for 24 hours and further at room temperature for 4 hours. The reaction mixture was added to a mixture of 103 ml of 6 N hydrochloric acid, 50 ml of ice water and 100 ml of diethyl ether. The resulting mixture was adjusted to pH 2.0 with 6 N hydrochloric acid. The organic layer was separated. The aqueous layer was extracted with 20 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was purified by column chromatography (eluant: toluene/ethyl acetate = 10/1) to obtain 6.3 g of colorless, oily 1-(1-naphthyl)-2-[2-(p-toluenesulfonyloxy)ethoxy]ethanol.

In the same manner, there was obtained colorless, oily 1-(2-naphthyl)-2-[2-(p-toluenesulfonyloxy)ethoxy]-ethanol.

(3) 0.82 g of pyridinium p-toluenesulfonate was added to a solution of 6.3 g of 1-(1-naphthyl)-2-[2-(p-toluenesulfonyloxy)ethoxy]ethanol and 2.97 ml of 3,4-dihydro-2H-pyran dissolved in 63 ml of methylene chloride at room temperature. The resulting mixture was stirred at the same temperature for 20 minutes and further at 35-40°C for 10 minutes. The reaction mixture was cooled, washed with water, and dried over anhydrous magnesium sulfate. The solvent was removed by distillation under reduced pressure. The residue thus obtained was purified by column chromatography (eluant: toluene/ethyl acetate = 10/1) to obtain 7.53 g of colorless, oily 1-(1-naphthyl)-1-(tetrahydropyran-2-yloxy)-2-[2-(p-toluenesulfonyloxy)ethoxy]ethane.

In the same manner, there was obtained colorless, oily 1-(2-naphthyl)-1-(tetrahydropyran-2-yloxy)-2-[2-(p-toluenesulfonyloxy)ethoxy]ethane.

(4) A mixture of 7.5 g of 1-(1-haphthyl)-1-(tetrahydropyran-2-yloxy)-2-[2-(p-toluenesulfonyloxy)-ethoxy]ethane, 2.65 ml of N-methylpiperazine, 3.96 g of potassium carbonate and 38 ml of N,N-dimethylformamide was stirred for 2 hours at 90-100°C. The reaction mixture was cooled and added to a mixture of 100 ml of diethyl ether and 100 ml of ice water. The organic layer was separated. The aqueous layer was extracted twice each with 25 ml of diethyl ether. The extracts were combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was purified by column chromatography (eluant: chloroform/ethanol = 10/1) to obtain 3.36 g of colorless, oily 2-[2-(4-methylpiperazin-1-yl) ethoxy]-1-(1-naphthyl)-1-(tetrahydropyran-2-yloxy)ethane (compound No. 31).

(5) In 30 ml of acetone was dissolved 3.3 g of 2-[2-(4-methylpiperazin-1-yl)ethoxy]-1-(1-naphthyl)-1-(tetrahydropyran-2-yloxy)ethane. To the solution were added 3.46 g of p-toluenesulfonic acid monohydrate and 7 ml of water at room temperature. The resulting mixture was stirred at the same temperature for 30 minutes and further at 40°C for 1 hour. The reaction mixture was added to a mixture of 60 ml of chloroform and 60 ml of ice water. The mixture was adjusted to pH 11 with a 10% aqueous sodium hydroxide solution. The organic layer was separated. The aqueous layer was extracted with 20 ml of chloroform. The extract was combined with the previously separated organic layer. The combined organic layer was washed with water and dried over anhydrous magnesium sulfate. The solvent was removed by distillation under reduced pressure. The residue thus obtained was dissolved in 40 ml of acetone. Hydrogen chloride gas was blown into the solution with ice cooling. The resulting solution was stirred at room temperature for 30 minutes. Thereto was added 40 ml of diethyl ether. The resulting mixture was stirred at the same temperature for 30 minutes. The resulting crystals were collected by filtration, washed with acetone, and dried to obtain 2.35 g of 2-[2-(4-methylpiperazin-1-yl)ethoxy]-1-(1-naphthyl)ethanol dihydrochloride (compound No. 32).

Melting point: 230.5-231.5°C [MeOH]

Production Example 4

The same procedure as in (4) and (5) of Production Example 3 was repeated, except that the N-methylpiperazine was replaced by N-(p-fluorobenzoyl)-piperidine, to obtain 2-{2-[4-(p-fluorobenzoyl)piperidin-1-yl]ethoxy}-1-(1-naphthyl)ethanol hydrochloride (compound No. 33).

Melting point: 204.5-205.5°C [MeOH]

Production Example 5

In 20 ml of ethanol was dissolved 2 g of 1-(1-naphthyl)-1-(tetrahydropyran-2-yloxy)-2-[2-(p-toluenesulfonyloxy) ethoxy]ethane. To the solution was added 6.60 g of a 40% aqueous methylamine solution at room temperature. The mixture was refluxed for 1 hour. The reaction mixture was cooled and added to a mixture of 20 ml of ice water and 50 ml of diethyl ether. The organic layer was separated. The aqueous layer was extracted with 20 ml of diethyl ether. The extract was combined with the previously separated organic layer. To the combined organic layer was added 15 ml of water and the resulting mixture was adjusted to pH 1.5 with 6 N hydrochloric acid. The aqueous layer was separated. The organic layer was extracted twice each with 10 ml of water. The extracts were combined with the previously separated aqueous layer. The combined aqueous layer was mixed with 25 ml of chloroform and adjusted to pH 11 with a 10% aqueous sodium hydroxide solution. The organic layer was separated. The aqueous layer was extracted twice each with 10 ml of chloroform. The extracts were combined with the previously separated organic layer. The combined organic layer was dried over anhydrous magnesium sulfate. The solvent was removed by distillation under reduced pressure to obtain 1.27 g of an oily product. The oily product was dissolved in 10 ml of acetone. Into the solution was blown hydrogen chloride gas with ice cooling. Thereto was added 10 ml of diethyl ether. The resulting crystals were collected by filtration to obtain 0.72 g of 2-[2-(N-methylamino)ethoxy]-1-(1-naphthyl)ethanol hydrochloride (compound No.34).

Melting point: 137.5-139°C [IPA]

The compounds shown in Table 6 were obtained in the same manner.

In Table 6, $R^1$, $R^2$, $R^3$, $R^{4a}$, $R^{4b}$, $R^6$, na and nb each show a substituent or integer used in the following formula:

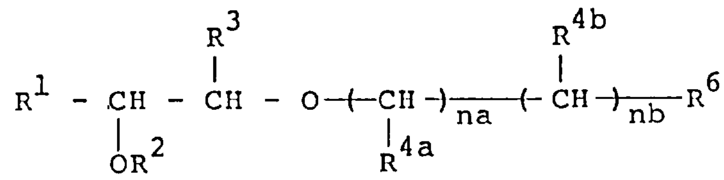

Table 6

| Compound No. | $R^1$ | $R^2$ | $R^3$ | $R^{4a}$ | $R^{4b}$ | $R^6$ | na | nb | Addition salt | Melting point (°C) |
|---|---|---|---|---|---|---|---|---|---|---|
| 35 | 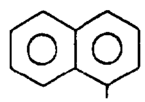 | H | H | H | H | 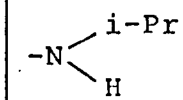 | 1 | 1 | HCl | 180-181 [EtOH] |
| 36 | " | " | " | " | " | 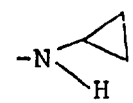 | " | " | - | 159.5-162 |
| 37 | " | " | " | " | " | 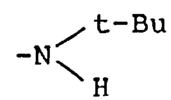 | " | " | 1/2 Maleic acid | 184-185 [IPA-AcOEt] |
| 38 | 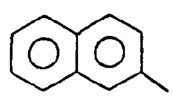 | " | " | " | " | 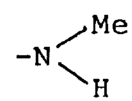 | " | " | HCl | 170-171 [EtOH] |
| 39 | " | " | " | " | " | 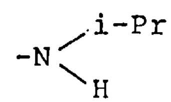 | " | " | " | 180.5-181.5 [IPA-EtOH] |

Production Example 6

A mixture of 3.5 g of potassium tert-butoxide, 9.4 g of N-tritylethanolamine and 50 ml of dimethyl sulfoxide was heater to 85°C. Thereto was added 5.3 g of 2-(l-naphthyl)oxirane. The resulting mixture was stirred at the same temperature for 5 minutes. The reaction mixture was cooled and added to a mixture of 100 ml of ethyl acetate and 150 ml of ice water. The organic layer was separated. The aqueous layer was extracted with 50 ml of ethyl acetate. The extract was combined with the previously separated organic layer. The combined organic layer was washed with water and a saturated aqueous sodium chloride solution in this order, and dried over anhydrous magnesium sulfate. The solvent was removed by distillation under reduced pressure. To the residue thus obtained were added 80 ml of a 50% aqueous formic acid solution and 40 ml of tetrahydrofuran. The resulting mixture was stirred at 50-60°C for 1 hour. The reaction mixture was cooled. The solvent was removed by distillation under reduced pressure. To the residue thus obtained were added 60 ml of ethyl acetate and 60 ml of water. The resulting mixture was adjusted to pH 2 with 6 N hydrochloric acid. The aqueous layer was separated. The organic layer was extracted twice each with 15 ml of water. The extracts were combined with the previously separated aqueous layer. To the combined aqueous layer was added 100 ml of chloroform and the resulting mixture was adjusted to pH 10.5 with potassium carbonate. The organic layer was separated, washed with water, and dried over anhydrous magnesium sulfate. The solvent was removed by distillation under reduced pressure. To the residue thus obtained was added 10 ml of diisopropyl ether. The resulting crystals were collected by filtration and dried to obtain 1.8 g of 2-(2-aminoethoxy)-1-(1-naphthyl)ethanol (compound No. 40).

Melting point: 89.5-92°C [$CHCl_3$-$Et_2O$]

Production Example 7

7 ml of water and 7 ml of dioxane were added to 0.7 g of 2-(2-aminoethoxy)-1-(1-naphthyl)-ethanol to obtain a solution. To the solution was added 0.32 g of potassium carbonate. The resulting mixture was heated to 50°C. Thereto was added 0.35 g of 2-chloropyrimidine. The resulting mixture was refluxed for 3 hours. Thereto were added 0.32 g of potassium carbonate and 0.35 g of 2-chloropyrimidine. The resulting mixture was further refluxed for 2.5 hours. The reaction mixture was cooled and added to a mixture of 20 ml of ethyl acetate and 20 ml of ice water. The organic layer was separated. The aqueous layer was extracted with 10 ml of ethyl acetate. The extract was combined with the previously separated organic layer. To the combined organic layer was added 15 ml of water and the resulting mixture was adjusted to pH 1.5 with 6 N hydrochloric acid. The aqueous layer was separated. The organic layer was extracted with 10 ml of water. The extract was combined with the previously separated aqueous layer. To the combined aqueous layer was added 50 ml of methylene chloride and the resulting mixture was adjusted to pH 10.5 with potassium carbonate. The organic layer was separated, washed with water, and dried over anhydrous magnesium sulfate. The solvent was removed by distillation under reduced pressure. The residue thus obtained was purified by column chromatography (eluant: chloroform/ethanol = 20/1) to obtain an oily product. To the oily product were added 4 ml of ethanol and 0.21 g of maleic acid. The resulting mixture was stirred at room temperature for 1 hour. To the reaction mixture was added 2 ml of diethyl ether. The resulting mixture was stirred at the same temperature for 1 hour. The resulting crystals were collected by filtration and dried to obtain 0.53 g of 1-(1-naphthyl)-2-{2-[(pyrimidin-2-yl)amino]ethoxy}-ethanol maleate (compound No. 41).

Melting point: 101.5-103°C [EtOH-AcOEt]

Production Example 8

0.62 g of N,N'-dicyclohexylcarbodiimide was added to a mixture of 0.7 g of 2-(2-aminoethoxy)-1-(1-naphthyl)ethanol, 0.37 g of nicotinic acid, 0.41 g of l-hydroxybenzotriazole, 0.42 ml of triethylamine and 4 ml of tetrahydrofuran with ice cooling. The resulting mixture was stirred at the same temperature for 5 minutes and further at room temperature for 1 hour. To the reaction mixture was added 6 ml of ethyl acetate. The insolubles were removed by filtration. To the filtrate were added 15 ml of ethyl acetate and 20 ml of water. The resulting mixture was adjusted to pH 2 with 6 N hydrochloric acid. The aqueous layer was separated. The organic layer was extracted twice each with 10 ml of water. The extracts were combined with the previsously separated aqueous layer. To the combined aqueous layer was added 30 ml of chloroform and the resulting mixture was adjusted to pH 10.5 with potassium carbonate. The organic layer was separated, washed with water, and dried over anhydrous magnesium sulfate. The solvent was removed by distillation under reduced pressure. The residue thus obtained was purified by column chromatography (eluant: chloroform/ethanol = 10/1) to obtain an oily product. The oily product was dissolved in 7 ml of acetone. To the solution was added 0.43 ml of a 5 N dry hydrogen chloride-ethanol solution. The resulting mixture was stirred at room temperature for 1 hour. To the reaction mixture was added 3 ml of diethyl ether. The resulting mixture was stirred at the same temperature for 1 hour. The resulting crystals were collected by filtration and dried to obtain 0.67 g of 1-(1-naphthyl)-2-[2-(nicotinoylamino)ethoxy]ethanol hydrochloride (compound no. 42).

Melting point: 162.5-163.5°C [EtOH-AcOEt]

Production Example 9

The same procedure as in (1) of Production Example 2 was repeated, except that the 2-(2-naphthyl)-oxirane and the 2-hydroxymethyl-4-tritylmorpholine were replaced by 2-(1-naphthyl)oxirane and 1,4-diformyl-2-piperazinyl methanol, respectively, to obtain oily 2-[(1,4-diformylpiperazin-2-yl)methoxy]-1-(1-naphthyl)-ethanol (compound No. 43).

The compounds shown in Table 7 were obtained in the same manner.

In Table 7, $R^1$, $R^2$, $R^3$, $R^4$, $R^6$ and n each show a substituent or integer used in the following general formula:

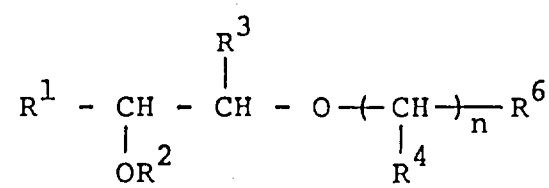

Table 7

| Compound No. | $R^1$ | $R^2$ | $R^3$ | $R^4$ | $R^6$ | n | Melting point (°C) |
|---|---|---|---|---|---|---|---|
| 44 | 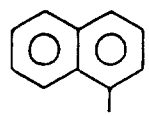 | H | H | H | 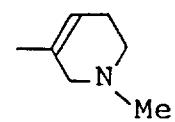 | 1 | 98-100 [AcOEt] |
| 45 | 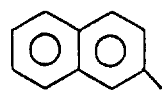 | " | " | " | 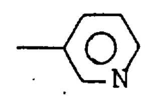 | " | 83-85 [AcOEt-IPE] |
| 46 | 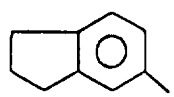 | " | " | " | " | " | 72-73.5 [AcOEt-IPE] |

Production Example 10

In 1.5 ml of methanol was dissolved 250 mg of 2-[(1,4-diformylpiperazin-2-yl)methoxy]-1-(1-naphthyl)-ethanol. To the solution was added 1.5 ml of a 5 N dry hydrogen chloride-ethanol solution. The mixture was allowed to stand at room temperature overnight. The resulting crystals were collected by filtration, washed with ethanol, and dried to obtain 180 mg of 1-(1-naphthyl)-2-[(piperazin-2-yl)methoxy]ethanol dihydrochloride (compound No. 47).

Melting point: 199-201°C (decomp.)

Production Example 11

The compound obtained in the same manner as in (1) of Production Example 2 was reacted with hydrogen chloride gas in the same manner as in the production of the hydrochloride of Production Example 8, to obtain the compounds shown in Table 8.

In Table 8, $R^1$, $R^2$, $R^3$, $R^4$, $R^6$ and n each show a substituent or integer used in the following general formula:

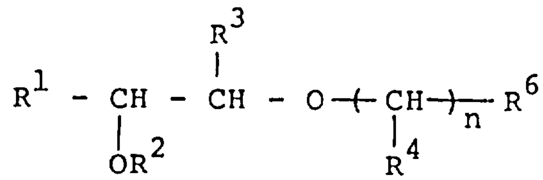

Table 8

| Compound No. | $R^1$ | $R^2$ | $R^3$ | $R^4$ | $R^6$ | n | Addition salt | Melting point (°C) |
|---|---|---|---|---|---|---|---|---|
| 48 | 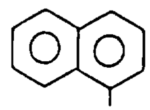 | H | H | H | 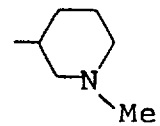 | 1 | HCl | 162-164 [EtOH-$Et_2O$] |
| 49 | " | " | " | " | 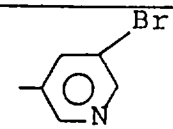 | " | " | 143-146 |
| 50 | " | " | " | - | 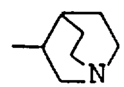 | 0 | " | 151.5-154 [EtOH-$Et_2O$] |
| 51 | " | " | " | H | 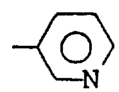 | 1 | " | 154-156 [EtOH-IPA] |

Production Example 12

The compounds shown in Table 9 were obtained in the same manner as in (1) and (2) of Production Example 2. In Table 9, $R^1$, $R^2$, $R^3$, $R^4$, $R^6$ and n each show a substituent or integer used in the following general formula:

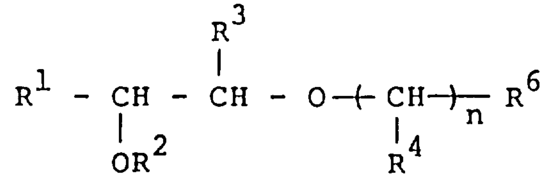

Table 9

| Compound No. | $R^1$ | $R^2$ | $R^3$ | $R^4$ | $R^6$ | n | Addition salt | Melting point (°C) |
|---|---|---|---|---|---|---|---|---|
| 52 | 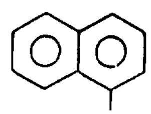 | H | H | H | 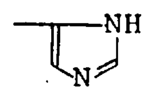 | 1 | - | Oily |
| 53 | " | " | " | " | " | " | 1/2 Fumaric acid | 167.5-170 [EtOH] |
| 54 | 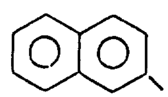 | " | " | " | " | " | - | 122-123 [$Me_2CO$-AcOEt] |
| 55 | 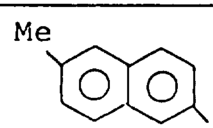 | " | " | " | " | " | - | 138-139 [$Me_2CO$-AcOEt] |

Production Example 13

2-(Imidazol-5-yl)methoxy-1-(1-naphthyl)-ethanol was reacted with methyl iodide to obtain oily 2-(1-methylimidazol-5-yl)methoxy-1-(1-naphthyl)ethanol (compound No. 56).

Production Example 14

(1) 6.0 g of 6-benzyloxy-2-naphthaldehyde was dissolved in 60 ml of tetrahydrofuran. The solution was cooled to -30°C. Thereto was dropwise added, in 10 minutes, 30 ml of a tetrahydrofuran solution containing 1.6 M of 2-chloroethoxymethylmagnesium chloride. The resulting mixture was stirred for 1 hour with ice cooling. The reaction mixture was added to a mixture of 100 ml of ice water, 100 ml of ethyl acetate and 3.6 g of ammonium chloride. The mixture was adjsuted to pH 2 with 6 N hydrochloric acid. The organic layer was separated, washed with water and a saturated aqueous sodium chloride solution in this order, and dried over anhydrous magnesium chloride. The solvent was removed by distillation under reduced pressure. The residue thus obtained was purified by column chromatography (eluant: toluene/ethyl acetate = 20/1) to obtain a solid. To the solid was added 10 ml of diisopropyl ether. The resulting mixture was stirred at room temperature for 1 hour. The resulting crystals were collected by filtration and dried to obtain 4.7 g of 1-(6-benzyloxy-2-naphthyl)-2-(2-chloroethoxy)ethanol.

Melting point: 86-87.5°C [IPE]

(2) A mixture of 4.5 g of 1-(6-benzyloxy-2-naphthyl)-2-(2-chloroethoxy)ethanol, 1 g of potassium iodide, 10 ml of a 50% aqueous dimethylamine solution and 20 ml of ethanol was refluxed for 3 hours. To the reaction mixture was added 10 ml of a 50% aqueous dimethylamine solution. The resulting mixture was further refluxed for 6 hours. The reaction mixture was cooled and concentrated to about a half volume under reduced pressure. To the concentrate were added 100 ml of ethyl acetate and 100 ml of water. The mixture was adjusted to pH 10.5 with potassium carbonate. The organic layer was separated, washed with water and a saturated aqueous sodium chloride solution in this order, and dried over anhydrous magnesium sulfate. The solvent was removed by distillation under reduced pressure. To the residue thus obtained was added 30 ml of diethyl ether. The resulting crystals were collected by filtration and dried to obtain 3.9 g of 1-(6-benzyloxy-2-naphthyl)-2-[2-(N,N-dimethylamino)ethoxy]-ethanol (compound No. 57).

Melting point: 100-100.5°C [EtOH-$H_2O$]

1-(6-Benzyloxy-2-naphthyl)-2-[2-(N,N-dimethyl-amino)ethoxy]ethanol was treated in the same manner as in Production Example 22, to obtain 1-(6-benzyloxy-2-naphthyl)-2-[2-(N,N-dimethylamino)ethoxy]ethanol hydrochloride (compound No. 58).

Melting point: 220-220.5°C [EtOH]

Production Example 15

In 12 ml of pyridine was suspended 3.0 g of 1-(6-benzyloxy-2-naphthyl)-2-[2-(N,N-dimethylamino)-ethoxy]ethanol. To the suspension was added 1.6 ml of acetic anhydride. The resulting mixture was stirred at room temperature for 24 hours. After the completion of the reaction, the solvent was removed by distillation under reduced pressure. To the residue thus obtained were added 60 ml of ethyl acetate and 60 ml of water. The mixture was adjusted to pH 10.5 with potassium carbonate. The organic layer was separated. The aqueous layer was extracted with 30 ml of ethyl acetate. The extract was combined with the previously separated organic layer. The combined organic-layer was washed with water and a saturated aqueous sodium chloride solution in this order, and dried over anhydrous magnesium sulfate. The solvent was removed by distillation under reduced pressure. The residue thus obtained was dissolved in 30 ml of acetone. To the solution was added 1.5 ml of a 5 N dry hydrogen chloride-ethanol solution. The resulting mixture was stirred at room temperature for 1 hour. The resulting cyrstals were collected by filtration and dried to obtain 2.6 g of 1-acetoxy-1-(6-benzyloxy-2-naphthyl)-2-[2-(N,N-dimethylamino)ethoxy]ethane hydrochloride (compound No. 59).

Melting point: 157-158°C [MeCN]

Production Example 16

A mixture of 2.0 g of 1-acetoxy-1-(6-benzyloxy-2-naphthyl)-2-[2-(N,N-dimethylamino)ethoxy]-ethane hydrochloride, 0.5 g of 5% palladium-carbon and 40 ml of ethanol was subjected to hydrogenation at room temperature under atmospheric pressure. After the completion of the reaction, the palladium-carbon was removed by filtration. The solvent was removed by distillation under reduced pressure. Acetone was added to the residue thus obtained. The resulting crystals were collected by filtration and dried to obtain 0.76 g of 1-acetoxy-1-(6-hydroxy-2-naphthyl)-2-[2-(N,N-dimethylamino)ethoxy]ethane hydrochloride (compound No. 60).

Melting point: 150.5-151.5°C [EtOH]

Production Example 17

A mixture of 360 mg of 1-acetoxy-1-(6-hydroxy-2-naphthyl)-2-[2-(N,N-dimethylamino)ethxoy]ethane hydrochloride, 10 ml of water and 15 ml of chloroform was adjusted to pH 9 with a saturated aqueous sodium hydrogencarbonate solution. The organic layer was separated. The aqueous layer was extracted twice each with 10 ml of chloroform. The extracts were combined with the previously separated organic layer. The combined organic layer was washed with saturated aqueous sodium chloride solution and dried over anhydrous magnesium sulfate. The solvent was removed by distillation under reduced pressure. The residue thus obtained was dissolved in 6 ml of benzene. To the solution was added 0.12 ml of ethyl isocyanate. The resulting mixture was stirred for 4 hours at 80°C. The reaction mixture was cooled. The solvent was removed by distillation under reduced pressure. The residue thus obtained was purified by column chromatography (eluant: chloroform/ethanol = 20/1) to obtain an oily product. The oily product was treated in the same manner as in Production Example 22 to obtain, as an amorphous, 150 mg of 1-acetoxy-1-[6-(N-ethylcarbamoyl)oxy-2-naphthyl]-2-[2-(N,N-dimethylamino)ethoxy]ethane hydrochloride (compound No. 61).

Production Example 18

2-[2-(N,N-dimethylamino)ethoxy]-1-(8-nitro-1-naphthyl)ethanol was obtained in the same manner as in (1) and (2) of Production Example 13. This compound was reacted with oxalic acid in the same manner as in (2) of Production Example 2 to obtain 2-[2-(N,N-dimethylamino)ethoxy]-1-(8-nitro-1-naphthyl)ethanol oxalate (compound No. 62).

Melting point: 150-158.5°C

Production Example 19

A mixture of 150 mg of 2-[2-(N,N-dimethyl-amino)ethoxy]-1-(8-nitro-1-naphthyl)ethanol oxalate, 150 mg of 5% palladium-carbon and 3 ml of methanol was subjected to hydrogenation at room temperature for 30 minutes under atmospheric pressure. After the completion of the reaction, the palladium-carbon was removed by filtration. The filtrate was subjected to distillation under reduced pressure to remove the solvent. The residue thus obtained was added to a mixture of 30 ml of ice water and 30 ml of chloroform. The mixture was adjusted to pH 11 with a 2 N aqueous sodium hydroxide solution. The organic layer was separated, washed with water and a saturated aqueous sodium chloride solution in this order, and dried over anhydrous magnesium sulfate. The solvent was removed by distillation under reduced pressure. The residue thus obtained was dissolved in 1 ml of ethanol. Thereto was added 0.2 ml of a 5.9 N dry hydrogen chloride-ethanol solution. The resulting crystals were collected by filtration and dried to obtain 110 mg of 1-(8-amino-1-naphthyl)-2-[2-(N,N-dimethylamino) ethoxy]ethanol dihydrochloride (compound No. 63).

Melting point: 195-198°C (decomp.) [AcOEt-EtOH]

Production Example 20

Triethylamine was added to 1-(8-amino-1-naphthyl)-2-[2-(N,N-dimethylamino)ethoxy]ethanol hydrochloride. The resulting mixture was reacted with methanesulfonyl chloride to obtain oily 2-[2-(N,N-dimethylamino)ethoxy]-1-(8-methylsulfonylamino-1-naphthyl)ethanol hydrochloride (compound No. 64).

Production Example 21

The same procedure as in (1) and (2) of Production Example 14 was repeated, except that the 6-benzyloxy-2-naphthaldehyde was replaced by 4-(N,N-dimethylamino)-1-naphthaldehyde, to obtain oily 1-[4-(N,N-dimethylamino)-1-naphthyl]-2-[2-(N,N-dimethylamino)ethoxy]ethanol dihydrochloride (compound No. 65).

Production Example 22

13 ml of ethanol was added to 1.3 g of 1-[4-(N, N-dimethylamino)-1-naphthyl]-2-[2-(N,N-dimethylamino)-ethoxy] ethanol dihydrochloride. The resulting mixture was refluxed for 1 hour. The reaction mixture was cooled. The solvent was removed by distillation under reduced pressure. To the residue thus obtained was added a mixture of 20 ml of ethyl acetate and 20 ml of water. The resulting mixture was adjusted to pH 10 with potassium carbonate. The organic layer was separated, washed with a saturated aqueous sodium chloride solution, and dried over anhydrous magnesium sulfate. The solvent was removed by distillation under reduced pressure to obtain 1.3 g of oily 1-ethoxy-1-[4-(N,N-dimethylamino)-l-naphthyl]-2-[2-(N,N-dimethyl-amino)ethoxy]ethane (compound No. 66).

Production Example 23

(1) 9.6 g of 5-bromo-1-hydroxyindane was dissolved in 100 ml of dry methylene chloride. To the solution were added, at room temperature, 570 mg of pyridinium p-toluenesulfonate and 4.5 ml of 3,4-dihydro-2H-pyran. The resulting mxiture was stirred at the same temperature for 3 hours. The reaction mixture was added to ice water. The organic layer was separated, washed with a saturated aqueous sodium chloride solution, and dried over anhydrous magnesium sulfate. The solvent was removed by distillation under reduced pressure. The residue thus obtained was purified by column chromatography (eluant: toluene/ethyl acetate = 15/1) to obtain 13.1 g of oily 5-bromo-1-(tetrahydropyran-2-yloxy)indane.

(2) 8.1 g of 5-bromo-1-(tetrahydropyran-2-yloxy)-indane was dissolved in 100 ml of anhdyrous tetrahydrofuran under a nitrogen atmosphere. To the solution was dropwise added 20 ml of a 1.5 N n-butyllithiumhexane solution at -65°C in 10 minutes. The resulting mixture was stirred at the same temperature for 5 minutes. Thereto was added 2.3 ml of anhydrous N,N-dimethylformamide. The reaction mixture was heated to room temperature and added to a mixture of 100 ml of ice water, 100 ml of diethyl ether and 2 g of ammonium chloride. The organic layer was separated, washed with water and a saturated aqueous sodium chloride solution in this order, and dried over anhydrous magnesium sulfate. The solvent was removed by distillation under reduced pressure. The residue thus obtained was purified by column chromatography (eluant: toluene/ ethyl acetate = 15/1) to obtain 6.5 g of 5-formyl-1-(tetrahydropyran-2-yloxy)indane.

(3) The same procedure as in (1) and (2) of Production Example 14 was repeated, except that the 6-benzyloxy-2-naphthaldehyde was replaced by 5-formyl-1-(tetrahydropyran-2-yloxy)indane, to obtain oily 2-[2-(N,N-dimethylamino)ethoxy]-1-[1-(tetrahydropyran-2-yloxy)indan-5-yl]ethanol (compound No. 67).

(4) A mixture of 2.5 g of 2-[2-(N,N-dimethyl-amino)ethoxy]-1-[1-(tetrahydropyran-2-yloxy)indan-5-yl]-ethanol, 8 ml of acetic anhydride and 0.64 ml of pyridine was stirred at room temperature for 1 hour. The reaction mixture was added to a mixture of 50 ml of ice water and 50 ml of diethyl ether. The organic layer was separated and dried over anhydrous magnesium sulfate. The solvent was removed by distillation under reduced pressure. The residue thus obtained was purified by column chromatography (eluant: chloroform/methanol = 10/1) to obtain 1.9 g of oily 1-acetoxy-2-[2-(N,N-dimethylamino)ethoxy]-1-[1-(tetrahydropyran-2-yloxy)-indan-5-yl]ethane (compound No. 68).

(5) 1.8 g of 1-acetoxy-2-[2-(N,N-dimethylamino)-ethoxy]-1-[1-(tetrahydropyran-2-yloxy)indan-5-yl]ethane was added to 30 ml of a 4:2:1 mixed solution of acetic acid, tetrahydrofuran and water. The resulting mixture was stirred at 70°C for 2 hours. The reaction mixture was cooled and added to a mixture of 100 ml of water and 100 ml of diethyl ether. The resulting mixture was adjusted to pH 8.5 with potassium carbonate. The organic layer was separated and dried over anhydrous magnesium sulfate. The solvent was removed by distillation under reduced pressure. The residue thus obtained was purified by column chromatography (eluant: chloroform/ethanol = 10/1) to obtain 1.0 g of oily 1-acetoxy-1-[(1-hydroxy)indan-5-yl]-2-[2-(N,N-dimethyl-amino)ethoxy]ethane (compound No. 69).

(6) In 5 ml of pyridine was dissolved 1.0 g of 1-acetoxy-1-[(1-hydroxy)indan-5-yl]-2-[2-(N,N-dimethylamino)-ethoxy] ethane. To the solution was added 0.3 ml of methanesulfonyl chloride at room temeprature. The resulting mixture was stirred at the same temperature overnight. The reaction mixture was added to a mixture of 50 ml of ice water and 50 ml of diethyl ether. The resulting mixture was adjusted to pH 8.5 with potassium carbonate. The organic layer was separated and dried over anhydrous magnesium sulfate. The solvent was removed by distillation under reduced pressure. The residue thus obtained was purified by column chromatography (eluant: chloroform/methanol = 10/1) to obtain 80 mg of oily 1-acetoxy-1-(1H-inden-6-yl)-2-[2-(N,N-dimethylamino)ethoxy]ethane(compound No. 70).

(7) In 0.5 ml of methanol was dissolved 80 mg of 1-acetoxy-1-(1H-inden-6-yl)-2-[2-(N,N-dimethylamino)-ethoxy] ethane. To the solution was added 80 mg of a 28% sodium methoxide-methanol solution with ice cooling. The resulting mixture was stirred at room temperature for 20 minutes. The solvent was removed by distillation under reduced pressure. The residue thus obtained was purified by column chromatography (eluant: chloroform/methanol = 20/1) to obtain a yellow oily product. 5 The oily product was dissolved in 0.1 ml of ethanol. To the solution was added 0.1 ml of a 5.9 N dry hydroger chloride-ethanol solution at room temperature. The resulting crystals were collected by filtration to obtain 20 mg of 1-(1H-inden-6-yl)-2-[2-(N,N-dimethyl-amino)ethoxy]ethanol hydrochloride (compound No. 71).

Melting point: 168-171°C (decomp.) [AcOEt-EtOH]

Production Example 24

In the same manner as in the production of hydrochloride in (5) of Production Example 23 and Production Example 8, 1-[(1-hydroxy)indan-5-yl]-2-[2-(N,N-dimethylamino)ethoxy]ethanol hydrochloride (compound No. 72) was obtained.

Melting point: 150-152°C [IPA]

Production Example 25

2-[2-(N,N-dimethylamino)ethoxy]-1-(1-naphthyl)ethanol hydrochloride was reacted with acetic anhydride in pyridine in the presence of triethylamine to obtain oily 1-acetoxy-2-[2-(N,N-dimethyl-amino)ethoxy]-1-(1-naphthyl)ethane (compound No. 73).

Production Example 26

The compound obtained from 2-[2-(6-methyl-naphthyl)]oxirane in the same manner as in (1) of Production Example 2 was reacted with hydrogen chloride gas in the same manner as in the production of hydrochloride in Production Example 8, to obtain oily 2-[2-(N-methyl-2,3-dihydropyridin-6H-5-yl)methoxy]-1-[2-(6-methylnaphthyl)]ethanol (compound No. 74).

Production Example 27

The compounds shown in Table 10 were obtained in the same manner as in (1) and (2) of Production Example 14. In Table 10, $R^1$, $R^2$, $R^3$, $R^4$, $R^6$ and n each show a substituent or integer used in the following formula:

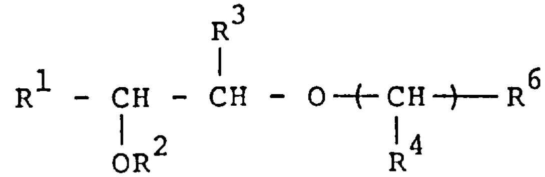

Table 10

| Compound No. | $R^1$ | $R^2$ | $R^3$ | $R^4$ | $R^6$ | n | Addition salt | Melting point (°C) |
|---|---|---|---|---|---|---|---|---|
| 75 | 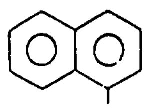 | H | H | H | 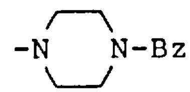 | 2 | 2HCl | 237-238 (decomp.) [EtOH-$H_2O$] |
| 76 | 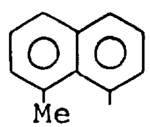 | " | " | " | 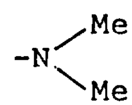 | " | HCl | 191-191.5 [IPA] |
| 77 | 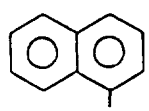 | " | " | " | 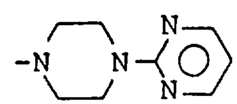 | " | 2HCl | 175-176.5 (decomp.) [EtOH] |
| 78 | " | " | " | " | 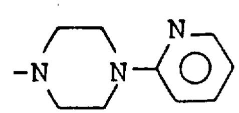 | " | " | 122-124 [EtOH] |

Table 10 (Cont'd)

| | | | | | | | | |
|---|---|---|---|---|---|---|---|---|
| 79 | 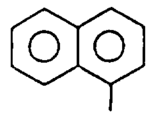 | H | H | H | 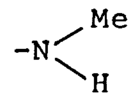 | 3 | - | 119-120 [AcOEt] |
| 80 | " | " | " | " | 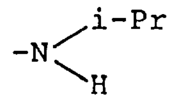 | " | HCl | 139-140.5 [EtOH-AcOEt] |
| 81 | " | " | " | " | 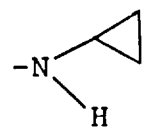 | " | " | 111.5-112 [EtOH-AcOEt] |
| 82 | 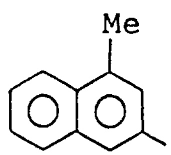 | " | " | " | 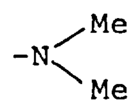 | 2 | " | 186-187 [EtOH-AcOEt] |

Table 10 (Cont'd)

| | | | | | | | | |
|---|---|---|---|---|---|---|---|---|
| 83 | 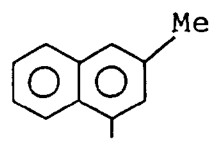 | H | H | H | 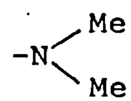 | 2 | HCl | 160-161.5 [EtOH-AcOEt] |
| 84 | 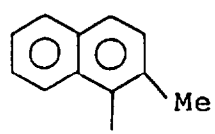 | " | " | " | " | " | " | 149-150 [EtOH-$Me_2CO$] |

Next, this invention is specifically described by way of Preparation Examples. However, this invention is in no way

restricted to these Examples.

Preparation Example 1 (Tablets)

Tablets each containing 50 mg of 2-[2-(N,N-dimethylamino)ethoxy]-1-(1-naphthy 1)ethanol-hydrochloride (compound No. 1) prepared using the following recipe according to the following method:

| Per tablet: | | |
|---|---|---|
| Compound No. 1 | 50 mg | ① |
| Lactose | 20 mg | ① |
| Kollidon CL (BASF's product) | 15 mg | ① |
| Corn starch | 30 mg | ① |
| AVICEL PH 101 (Asahi Kasei's product) | 50 mg | ① |
| Polyvinylpyrrolidone K-90 | 5 mg | |
| Light silica | 3 mg | ② |
| Magnesium stearate | 2 mg | ② |
| Total | 175 mg | |

The components were ① kneaded with an aqueous solution containing 8% of Polyvinylpyrrolidone K-90. The kneaded product was dried at 40°C and mixed with the components ②. The resulting mixture was made into round tablets each weighing 175 mg and having a diameter of 8 mm.

Preparation Example 2 (Capsules)

Capsules each containing 50 mg of 2-[2-(N,N-dimethylamino)ethoxy]-1-(naphthy 1)ethanol hydrochloride (compound No. 1) were prepared using the following recipe according to the following method:

Per capsule:

| | | |
|---|---|---|
| Compound No. 1 | 50 mg | ① |
| Lactose | 20 mg | ① |
| Corn starch | 53 mg | ① |
| Kollidon CL (BASF's product) | 2 mg | ① |
| Polyvinylpyrrolidone K-90 | 5 mg | |
| AVICEL PH 302 (Asahi Kasei's product) | 18 mg | ② |
| Magnesium stearate | 2 mg | ② |
| Total | 150 mg | |

The components ① were kneaded with an aqueous solution containing 8% of Polyvinylpyrrolidone K-90. The kneaded product was dried at 40°C and mixed with the components ②. The resulting mixture was charged into No. 3 gelatin capsules in an amount of 150 mg per capsule to obtain capsules.

Preparation Example 3 (Tablets)

2-[2-(N,N-dimethylamino)ethoxy]-1-(2-naphthyl)-ethanol hydrochloride (compound No. 2) was processed as in Preparation Example 1 to obtain tablet containing 50 mg of the compound.

Preparation Example 4 (Capsules)

2-[2-(N,N-dimethylamino)ethoxy]-1-(2-naphthyl)ethanol hydrochloride (compound No. 2) was processed as in Preparation Example 2 to obtain capsule containing 50 mg of the compound.

## Claims

1. A 1,2-ethanediol derivative represented by the following general formula or a salt thereof:

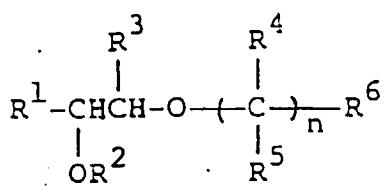

wherein $R^1$ represents a substituted or unsubstituted naphthyl, indanyl, indenyl or tetrahydronaphthyl group; $R^2$ represents a hydrogen atom, a $C_1$-$C_6$ alkyl group or a hydroxyl-protecting group; $R^3$ represents a hydrogen atom or a $C_1$ - $C_6$ alkyl group; $nR^4$'s and $nR^5$'s are the same as or different from one another and represent hydrogen atoms or $C_1$-$C_6$ alkyl groups; $R^6$ represents an ammonio group or a substituted or unsubstituted amino or nitrogen-containing heterocyclic group, the nitrogen-containing heterocyclic group being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6; wherein the substituent on $R^1$ is selected from the group consisting of halogen atoms, substituted or unsubstituted amino, $C_1$-$C_6$ alkyl, aryl, ar-$C_1$-$C_4$ alkyl, $C_1$-$C_6$ alkoxy, ar-$C_1$-$C_4$ alkoxy, aryloxy, carbamoyloxy, $C_1$-$C_6$ alkylthio, $C_2$-$C_6$ alkenyl, $C_2$-$C_6$ alkenyloxy, ar-$C_1$-$C_4$ alkylthio, ar-$C_1$-$C_6$ alkylsulfonyl, arylsulfonyl, $C_1$-$C_6$ alkylsulfonylamino, arylsulfonylamino and heterocyclic groups, protected amino groups, protected or unprotected hydroxyl groups, nitro group, oxo group and $C_1$-$C_4$ alkylenedioxy groups; the substituted $C_1$-$C_6$ alkyl, aryl, ar-$C_1$-$C_4$ alkyl, $C_1$-$C_6$ alkoxy, ar-$C_1$-$C_4$ alkoxy, aryloxy, carbamoyloxy, $C_1$-$C_6$ alkylthio, $C_2$-$C_6$ alkenyl, $C_2$-$C_6$ alkenyloxy, ar-$C_1$-$C_4$ alkylthio, ar-$C_1$-$C_6$ alkylsulfonyl, arylsulfonyl, $C_1$-$C_6$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted $C_1$-$C_6$ alkyl groups, $C_1$-$C_6$ alkyl groups substituted by a protected or unprotected hydroxyl group, unsubstituted or halogen-substituted aryl groups, unsubstituted or halogen-substituted aroyl groups, unsubstituted $C_1$-$C_6$ alkoxy groups, $C_1$-$C_6$ alkoxy groups substituted by a $C_1$-$C_6$ alkoxy group, $C_1$-$C_6$ acyl groups, ar-$C_1$-$C_4$ alkyl groups, ar-$C_2$-$C_6$ alkenyl groups, heterocyclic groups, heterocyclic-CO- groups, oxo group, $C_1$-$C_6$ alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted $C_1$-$C_6$ alkyl groups, $C_1$-$C_6$ alkyl groups substituted by a protected or unprotected carboxyl or hydroxyl group, cycloalkyl groups, aryl groups, $C_1$-$C_6$ acyl groups, ar-$C_1$-$C_4$ alkyl groups, heterocyclic groups, unsubstituted or oxo-substituted heterocyclic-CO- groups, adamantyl group, $C_1$-$C_6$ alkylsulfonyl groups and arylsulfonyl groups;

all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl and isoquinolyl groups.

2. A 1,2-ethanediol derivative or a salt thereof according to Claim 1, wherein $R^1$ represents a substituted or unsubstituted naphthyl group; $R^2$ represents a hydrogen atom or a hydroxyl-protecting group; $nR^4$'s are the same as or different from one another and represent hydrogen atoms or $C_1$-$C_6$ alkyl groups; $nR^5$'s represent hydrogen atoms; and $R^6$ represents an ammonio group or a substituted or unsubstituted amino or nitrogen-containing heterocyclic group, the nitrogen-containing heterocyclic group being selected from the group consisting of pyrrolyl, pyrrolidinyl, piperidinyl, piperazinyl, imidazolyl, pyrazolyl, pyridyl, pyrimidinyl, morpholinyl, quinolyl, quinolizinyl, tetrahydroquinolinyl, quinuclidinyl, thiazolyl and thiadiazolyl groups.

3. A 1,2-ethanediol derivative or a salt thereof according to Claim 1, wherein $R^1$ represents a naphthyl, indanyl or tetrahydronaphthyl group which may optionally be substituted by a halogen atom or a $C_1$-$C_6$ alkyl group; $R^2$ represents a hydrogen atom; $R^3$ represents a hydrogen atom; $nR^4$'s and $nR^5$'s represent hydrogen atoms; $R^6$ represents a morpholinyl group which has a free valence on a carbon atom forming the ring, a pyridyl group, an imidazolyl group, a phenyl-$C_{1-4}$alkyl group-substituted piperazinyl group or an amino group which may optionally be substituted by a $C_1$-$C_6$ alkyl or cycloalkyl group; and n represents an integer of 1 to 4.

4. 2-(Imidazolylmethoxy)-1-(1-naphthyl)ethanol or salt thereof.

5. 2-(1-Methyl-5-imidazolylmethoxy)-1-(1-naphthyl)-ethanol or a salt thereof.

6. A process for producing a 1,2-ethanediol derivative represented by the following general formula or a salt thereof:

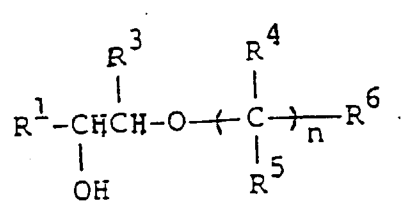

wherein $R^1$, $R^3$, $R^4$, $R^5$, $R^6$ and n are as defined in Claim 1, which comprises reacting a compound represented by the general formula:

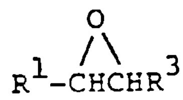

wherein $R^1$ and $R^3$ have the same meanings as defined in Claim 1 with a compound represented by the following general formula or a salt thereof:

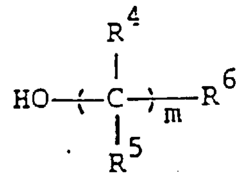

wherein $mR^4$'s and $mR^5$'s are the same as or different from one another and represent hydrogen atoms or lower, alkyl groups, $R^6$ has the same meaning as defined in Claim 1 and m represents an integer of 1 to 6, or a compound represented by the following general formula or a Salt thereof:

$$HO-R^{6a}$$

wherein $R^{6a}$ represents the same substituted or unsubstituted nitrogen-containing heterocyclic group as in the defintion of $R^6$, provided that the heterocyclic group has a free valence on a carbon atom forming the heterocyclic ring.

7. A process for producing a 1,2-ethanediol derivative represented by the following general formula or a salt thereof:

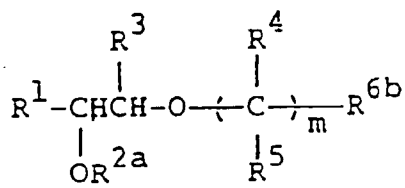

wherein $R^1$ and $R^3$ are as defined in Claim 1.

$R^{2a}$ represents a hydroxyl-protecting group;
$mR^4$'s and $mR^5$'s are the same as or different from one another and represent hydrogen atoms or $C_1$-$C_6$ alkyl groups; $R^{6b}$ represents a substituted or unsubstituted amino or nitrogen-containing heterocyclic group, the nitrogen-containing heterocyclic group having a free valence on a carbon atom forming the heterocyclic ring and being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and m represents an integer of 1 to 6, wherein the substituted nitrogen-containing heterocyclic group as $R^{6b}$ have each at least one substituent selected from the group consisting of halogen atoms, protected or unprotected hydroxyl groups, protected or unprotected amino groups, protected or unprotected carboxyl groups, unsubstituted $C_1$-$C_6$ alkyl groups, $C_1$-$C_6$ alkyl groups substituted by a protected or unprotected hydroxyl group, unsubstituted or halogen-substituted aryl groups, unsubstituted or halogen-substituted aroyl groups, unsubstituted $C_1$-$C_6$ alkoxy groups $C_1$-$C_6$ alkoxy groups substituted by a $C_1$-$C_6$ alkoxy group, $C_1$-$C_6$ acyl groups, ar-$C_1$-$C_4$ alkyl groups, ar-$C_2$-$C_6$ alkenyl groups, het-

erocyclic groups, heterocyclic-CO-groups, oxo group, $C_1$-$C_6$ alkylsulfonyl groups and arylsulfonyl groups; and'the substituted amino group as $R^{6b}$ have each at least one substituent selected from the group consisting of protected or unprotected hydroxyl groups, unsubstituted $C_1$-$C_6$ alkyl groups, $C_1$-$C_6$ alkyl groups substituted by a protected or unprotected carboxyl or hydroxyl group, cycloalkyl groups, aryl groups, $C_1$-$C_6$ acyl groups, ar-$C_1$-$C_4$ alkyl groups, heterocyclic groups, unsubstituted or oxo-substituted heterocyclic-CO- groups, adamantyl group, $C_1$-$C_6$ alkylsulfonyl groups and arylsulfonyl groups;

all the above heterocyclic groups being selected from the group consisting of the nitrogen-containing heterocyclic groups mentioned in the definition of $R^{6b}$ and furyl, thienyl, benzothienyl, pyranyl, isobenzofuranyl, oxazolyl, benzofuranyl, indolyl, benzimidazolyl; benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl and isoquinolyl groups, which comprises reacting a compound represented by the general formula:

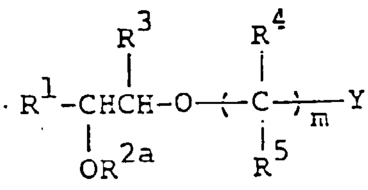

wherein $R^1$, $R^{2a}$, $R^3$, $R^4$, $R^5$ and m have the same meanings as defined above and Y represents a removable group, with a compound represented by the following general formula or a salt thereof:

$$H\text{-}R^{6b}$$

wherein $R^{6b}$ has the same meaning as defined above.

**8.** Use of a 1,2-ethanediol derivative represented by the following general formula or a salt thereof for preparing a drug for treating cerebrovascular dementia, senile dementia, Alzheimer's dementia, sequelae of ischemic encephalopathy or cerebral apoplexy in a patient:

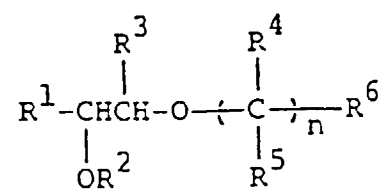

wherein $R^1$, $R^2$, $R^3$, $R^4$, $R^5$, $R^6$ and n are as defined in Claim 1.

**9.** Use of a 1,2-ethanediol derivative or a salt thereof according to Claim 8, wherein $R^1$ represents a substituted or unsubstituted naphthyl group; $R^2$ represents a hydrogen atom or a hydroxyl-protecting group; n$R^4$'s are the same as or different from one another and represent hydrogen atoms or $C_1$-$C_6$ alkyl groups; n$R^5$'s represent hydrogen atoms; and $R^6$ represents an ammonio group or a substituted or unsubstituted amino or nitrogen-containing heterocyclic group, the nitrogen-containing heterocyclic group being selected from the group consisting of pyrrolyl, pyrrolidinyl, piperidinyl, piperazinyl, imidizolyl, pyrazolyl, pyridyl, pyrimidinyl, morpholinyl, quinolyl, quinolizinyl, tetrahydroquinolinyl, quinuclidinyl, thiazolyl and thiadiazolyl groups.

**10.** Use of a 1,2-ethanediol derivative or a salt thereof according to Claim 8, wherein $R^1$ represents a substituted or unsubstituted naphthyl, indanyl or tetrahydronaphthyl group; $R^6$ represents an ammonio group or a substituted or unsubstituted amino or nitrogen-containing heterocyclic group, the nitrogen-containing heterocyclic group being selected from the group consisting of pyrrolinyl, piperidyl, piperazinyl, imidazolyl, pyridyl, morpholinyl, thiomorpholinyl, tetrahydropyridyl, quinuclidinyl and tetrazolyl, the substituent on $R^1$ is selected from the group consisting

of halogen atoms; a $C_1$-$C_6$ alkyl groups; $C_2$-$C_6$ alkenyl groups; phenyl groups which may optionally be substituted by a halogen atom or a $C_1$-$C_6$ alkyl or $C_1$-$C_6$ alkoxy group; phenyl-$C_{1-4}$alkyl groups; $C_1$-$C_6$ alkoxy groups which may optionally be substituted by a pyridyl, furyl or thienyl group; phenyl-$C_{1-4}$ alkoxy groups which may optionally be substituted by a halogen atom or a $C_1$-$C_6$ alkyl, hydroxyl or $C_1$-$C_6$ alkoxy group; a phenoxy group; phenylaminocarbonyloxy groups which may optionally be substituted by a halogen atom; $C_1$-$C_6$ alkylaminocarbonyloxy groups; $C_1$-$C_6$ alkylthio groups; a phenylsulfonylamino group; amino groups which may optionally be substituted by a $C_1$-$C_6$ alkyl group; a hydroxyl group; a nitro group; an oxo group; phenyl$C_{1-4}$alkylthio groups; phenyl-$C_{1-4}$alkylsulfonyl groups; a thienyl group; a pyridyl group; and $C_1$-$C_4$ alkylenedioxy groups; the substituent on $R^6$, when $R^6$ is a substituted amino group, is selected from the group consisting of $C_1$-$C_6$ alkyl groups which may optionally be substituted by a hydroxyl group; $C_1$-$C_6$ acyl groups; cycloalkyl groups; phenyl $C_{1-4}$ alkyl groups; a pyrimidinyl group; a pyridinecarbonyl group and an adamantyl group; and the substituent on $R^6$, when $R^6$ is a substituted nitrogen-containing heterocyclic group, is selected from the group consisting of halogen atoms; a hydroxyl group; $C_1$-$C_6$ alkyl groups which may optionally be substituted by a hydroxyl group; an amino group; $C_1$-$C_6$ acyl groups which may optionally be substituted by a pyrrolidinyl group; phenyl-$C_{1-4}$ alkyl groups; phenyl-$C_{2-4}$ alkenyl groups; aroyl groups which are substituted by a halogen atom; a pyridyl group and an oxo group.

**11.** Use of a 1,2-ethanediol derivative or a salt thereof according to Claim 10, wherein $R^1$ represents a naphthyl, indanyl or tetrahydronaphthyl group which may optionally be substituted by a halogen atom or a $C_1$-$C_6$ alkyl group; $R^2$ represents a hydrogen atom; $R^3$ represents a hydrogen atom; $nR^4$'s and $nR^5$'s represent hydrogen atoms; $R^6$ represents a morpholinyl group which has a free valence on a carbon atom forming the ring, a pyridyl group, an imidazolyl group, a phenyl-$C_{1-4}$alkyl group-substituted piperazinyl group or an amino group which may optionally be substituted by a $C_1$-$C_6$ alkyl group or a cycloalkyl group; and n represents an integer of 1 to 4.

**12.** Use of a 1,2-ethanediol derivative or a salt thereof according to Claim 8 , wherein the derivative or salt is 2-(imidazolylmethoxy)-1-(1-naphthyl)ethanol or a salt thereof.

**13.** Use of a 1,2-ethanediol derivative or a salt thereof according to Claim 8 , wherein the derivative or salt is 2-(1-methyl-5-imidazolylmethoxy)-1-(1-naphthyl)ethanol or a salt thereof.

**14.** A cerebral function-improving agent comprising a 1,2-ethanediol derivative represented by the following general formula or a salt thereof:

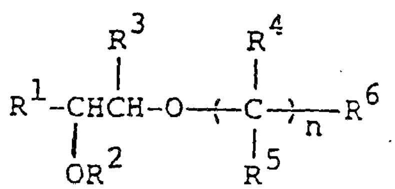

wherein $R^1$, $R^2$, $R^3$, $R^4$, $R^5$, $R^6$ and n are as defined in Claim 1.

**Patentansprüche**

**1.** 1,2-Ethandiol-Derivat, das durch die folgende allgemeine Formel dargestellt wird, oder ein Salz desselben:

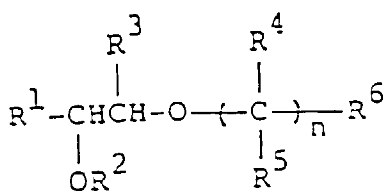

in der $R^1$ eine substituierte oder unsubstituierte Naphthyl-, Indanyl-, Indenyl- oder Tetrahydronaphthyl-Gruppe darstellt; $R^2$ ein Wasserstoffatom, eine ($C_1$-$C_6$)-Alkylgruppe oder eine Hydroxyl-Schutzgruppe darstellt; $R^3$ ein

Wasserstoffatom oder eine ($C_1$-$c_6$)-Alkylgruppe darstellt; die $nR^4$ und $nR^5$ gleich oder voneinander verschieden sind und Wasserstoffatome oder ($c_1$-$C_6$)-Alkylgruppen darstellen; $R^6$ eine Ammoniogruppe oder eine substituierte oder unsubstituierte Amino- oder Stickstoff-haltige heterocyclische Gruppe darstellt, wobei die Stickstoff-haltige heterocyclische Gruppe aus der Gruppe ausgewählt ist, die aus Pyrrolyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, chinuklidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolyl-Gruppen besteht; und n 0 oder eine ganze Zahl von 1 bis 6 darstellt; worin der Substituent an $R^1$ aus der Gruppe ausgewählt ist, die aus Halogenatomen, substituierten oder unsubstituierten Amino-, ($C_1$-$C_6$)-Alkyl-, Aryl-, Ar-($C_1$-$C_4$)-alkyl-, ($C_1$-$C_6$)-Alkoxy-, Ar-($C_1$-$C_4$)-alkoxy-, Aryloxy-, Carbamoyloxy-, ($C_1$-$C_6$) -Alkylthio-, ($C_2$-$C_6$)-Alkenyl-, ($C_2$-$C_6$) -Alkenyloxy-, Ar-($C_1$-$C_4$)-alkylthio-, Ar-($C_1$- $C_6$)-alkylsulfonyl-, Arylsulfonyl-, ($C_1$- $C_6$)-Alkylsulfonylamino-, Arylsulfonylamino- und heterocyclischen Gruppen, geschützten Aminogruppen, geschützten oder ungeschützten Hydroxylgruppen, der Nitrogruppe, Oxogruppe und ($C_1$-$C_4$)-Alkylendioxy-Gruppen besteht; wobei die substituierte ($C_1$-$C_6$)-Alkyl-, Aryl-, Ar-($C_1$-$C_4$)-alkyl-, ($C_1$-$C_6$)-Alkoxy-, Ar-($C_1$-$C_4$)-alkoxy-, Aryloxy-, Carbamoyloxy-, ($C_1$-$C_6$)-Alkylthio-, ($C_2$-$C_6$)-Alkenyl-, ($C_2$-$C_6$) -Alkenyloxy-, Ar-($C_1$-$C_4$)-alkylthio-, Ar-($C_1$-$C_6$)-alkylsulfonyl-, Arylsulfonyl-, ($C_1$-$C_6$)-Alkylsulfonylamino-, Arylsulfonylamino- oder hetercyclische Gruppe als Substituent von $R^1$ und die substituierte Stickstoff-haltige heterocyclische Gruppe als $R^6$ jeweils mindestens einen Substituenten aufweisen, der aus der Gruppe ausgewählt ist, die aus Halogenatomen, geschützten oder ungeschützten Hydroxylgruppen, geschützten oder ungeschützten Aminogruppen, geschützten oder ungeschützten Carboxylgruppen, unsubstituierten ($C_1$-$C_6$)-Alkylqruppen, ($C_1$-$C_6$)-Alkylgruppen, die durch eine geschützte oder ungeschützte Hydroxylgruppe substituiert sind, unsubstituierten oder Halogen-substituierten Arylgruppen, unsubstituierten oder Halogen-substituierten Aroylgruppen, unsubstituierten ($C_1$-$C_6$)-Alkoxygruppen, ($C_1$-$C_6$)-Alkoxygruppen, die durch eine ($C_1$-$C_6$)-Alkoxygruppe substituiert sind, ($C_1$-$C_6$)-Acylgruppen, Ar-($C_1$-$C_4$)-alkylgruppen, Ar-($C_2$-$C_6$)-alkenylgruppen, heterocyclischen Gruppen, Heterocyclus-CO-Gruppen, Oxogruppen, ($C_1$-$C_6$)-Alkylsulfonylgruppen und Arylsulfonylgruppen besteht; und die substituierte Aminogruppe als Substituent von $R^1$ und die substituierte Aminogruppe als $R^6$ jeweils mindestens einen Substituenten aufweisen, der aus der Gruppe ausgewählt ist, die aus geschützten oder ungeschützten Hydroxylgruppen, unsubstituierten ($C_1$-$C_6$) -Alkylgruppen, ($C_1$-$C_6$)-Alkylgruppen, die durch eine geschützte oder ungeschützte Carboxyl- oder Hydroxylgruppe substituiert sind, cycloalkylgruppen, Arylgruppen, ($C_1$-$C_6$)-Acylgruppen, Ar-($C_1$-$C_4$) -alkylgruppen, heterocyclischen Gruppen, unsubstituierten oder Oxo-substituierten heterocyclischen-CO-Gruppen, einer Adamantylgruppe, ($C_1$-$C_6$)-Alkylsulfonylgruppen und Arylsulfonylgruppen besteht;
wobei alle obigen heterocyclischen Gruppen aus der Gruppe ausgewählt sind, die aus den in der Definition von $R^6$ erwähnten Stickstoff-haltigen heterocyclischen Gruppen und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl- , Benzo[b]piperazinyl- , chromenyl-, Isothiazolyl-, Isoxazolyl-, Oxadiazolyl-, Pyridazinyl-, Isoindolyl- und Isochinolyl-Gruppen besteht.

**2.** 1,2-Ethandiol-Derivat oder ein Salz desselben nach Anspruch 1, in dem $R^1$ eine substituierte oder unsubstituierte Naphthylgruppe darstellt; $R^2$ ein Wasserstoffatom oder eine Hydroxyl-Schutzgruppe darstellt; die $nR^4$ gleich oder voneinander verschieden sind und Wasserstoffatome oder ($C^1$-$C^6$)-Alkylgruppen darstellen; die $nR^5$ Wasserstoffatome darstellen; und $R^6$ eine Ammoniogruppe oder eine substituierte oder unsubstituierte Amino- oder Stickstoff-haltige heterocyclische Gruppe darstellt, wobei die Stickstoff-haltige hetercyclische Gruppe aus der Gruppe ausgewählt ist, die aus Pyrrolyl-, Pyrrolidinyl-, Piperidinyl-, Piperazinyl-, Imidazolyl-, Pyrazolyl-, Pyridyl-, Pyrimidinyl-, Morpholinyl-, Chinolyl-, Chinolizinyl-, Tetrahydrochinolinyl-, Chinuklidinyl-, Thiazolyl- und Thiadiazolyl-Gruppen besteht.

**3.** 1,2-Ethandiol-Derivat oder ein Salz desselben nach Anspruch 1, in dem $R^1$ eine Naphthyl-, Indanyl- oder Tetrahydronaphthyl-Gruppe darstellt, die gegebenenfalls durch ein Halogenatom oder eine ($C_1$-$C_6$)-Alkylgruppe substituiert sein kann; $R^2$ ein Wasserstoffatom darstellt; $R^3$ ein Wasserstoffatom darstellt; die $nR^4$ und $nR^5$ Wasserstoffatome darstellen; $R^6$ eine Morpholinyl-Gruppe darstellt, die an einem Kohlenstoffatom, das den Ring bildet, eine freie Valenz aufweist, eine Pyridylgruppe, eine Imidazolylgruppe, eine durch eine Phenyl-($C_1$-$C_4$)-alkylgruppe substituierte Piperazinylgruppe oder eine Aminogruppe, die gegebenenfalls durch eine ($C_1$-$C_6$)-Alkyl-oder Cycloalkylgruppe substituiert sein kann; und n eine ganze Zahl von 1 bis 4 darstellt.

**4.** 2-(Imidazolylmethoxy)-1-(1-naphthyl) ethanol oder ein Salz desselben.

**5.** 2-(1-Methyl-5-imidazolylmethoxy)-1-(1-naphthyl)ethanol oder ein Salz desselben.

**6.** Verfahren zur Herstellung eines 1,2-Ethandiol-Derivats, das durch die folgende allgemeine Formel dargestellt wird, oder eines Salzes desselben:

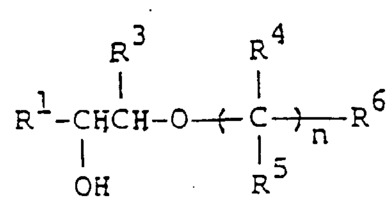

in der $R^1$, $R^3$, $R^4$, $R^5$, $R^6$ und n wie in Anspruch 1 definiert sind, umfassend das Umsetzen einer Verbindung, die durch die allgemeine Formel:

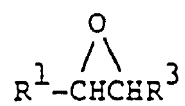

dargestellt wird, in der $R^1$ und $R^3$ die gleichen Bedeutungen, wie in Anspruch 1 definiert, aufweisen, mit einer Verbindung, die durch die folgende allgemeine Formel definiert wird, oder einem Salz derselben:

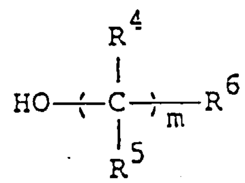

in der die $mR^4$ und $mR^5$ gleich oder voneinander verschieden sind und Wasserstoffatome oder Niederalkylgruppen darstellen, $R^6$ die gleiche Bedeutung, wie in Anspruch 1 definiert, aufweist und m eine ganze Zahl von 1 bis 6 darstellt, oder
mit einer Verbindung, die durch die folgende allgemeine Formel dargestellt wird, oder einem Salz derselben:

$$HO\text{-}R^{6a}$$

in der $R^{6a}$ die gleiche substituierte oder unsubstituierte Stickstoff-haltige heterocyclische Gruppe wie in der Definition von $R^6$ darstellt, vorausgesetzt, daß die heterocyclische Gruppe eine freie Valenz an einem Kohlenstoffatom aufweist, das den heterocyclischen Ring bildet.

**7.** Verfahren zur Herstellung eines 1,2-Ethandiol-Derivats, das durch die folgende allgemeine Formel dargestellt wird, oder eines Salzes desselben:

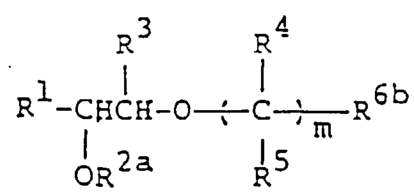

in der $R^1$ und $R^3$ wie in Anspruch 1 definiert sind, $R^{2a}$ eine Hydroxyl-Schutzgruppe darstellt; die $mR^4$ und $mR^5$ gleich oder voneinander verschieden sind und Wasserstoffatome oder $(C_1\text{-}C_6)$-Alkylqruppen darstellen; $R^{6b}$ eine substituierte oder unsubstituierte Amino- oder Stickstoff-haltige heterocyclische Gruppe darstellt, wobei die Stickstoff-haltige heterocyclische Gruppe eine freie Valenz an einem Kohlenstoff aufweist, das den heterocyclischen Ring bildet, und aus der Gruppe ausgewählt ist, die aus Pyrrolyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuklidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolyl-Gruppen besteht; und m eine ganze Zahl von 1 bis 6 darstellt, worin die substituierte Stickstoff-haltige heterocyclische Gruppe als $R^{6b}$ jeweils mindestens einen Substituenten aufweist, der aus der Gruppe ausgewählt ist, die aus Halogenatomen, geschützten oder ungeschützten Hydroxylgruppen, geschützten oder ungeschützten Aminogruppen, geschützten oder ungeschützten Carboxylgruppen, unsubstituierten ($C_1$-$C_6$)-Alkylgruppen, ($C_1$-$C_6$)-Alkylgruppen, die durch eine geschützte oder ungeschützte Hydroxylgruppe substituiert sind, unsubstituierten oder Halogen-substituierten Arylgruppen, unsubstituierten oder Halogen-substituierten Aroylgruppen, unsubstituierten ($C_1$-$C_6$)-Alkoxygruppen, ($C_1$-$C_6$)-Alkoxygruppen, die durch eine ($C_1$-$C_6$)-Alkoxygruppe substituiert sind, ($C_1$-$C_6$)-Acylgruppen, Ar-($C_1$-$C_4$)-alkylgruppen, Ar-($C_2$-$C_6$)-alkenylgruppen, heterocyclischen Gruppen, Heterocyclus-CO-Gruppen, einer Oxogruppe, ($C_1$-$C_6$)-Alkylsulfonylgruppen und Arylsulfonylgruppen besteht; und die substituierte Aminogruppe als $R^{6b}$ jeweils mindestens einen Substituenten aufweist, der aus der Gruppe ausgewählt ist, die aus geschützten oder ungeschützten Hydroxylgruppen, unsubstituierten ($C_1$-$C_6$)-Alkylgruppen, ($C_1$-$C_6$)-Alkylgruppen, die durch eine geschützte oder ungeschützte Carboxyl- oder Hydroxylgruppe substituiert sind, Cycloalkylgruppen, Arylgruppen, ($C_1$-$C_6$) -Acylgruppen, Ar-($C_1$-$C_4$)-alkylgruppen, heterocyclischen Gruppen, unsubstituierten oder Oxo-substituierten Heterocyclus-CO-Gruppen, der Adamantylgruppe, ($C_1$-$C_6$)-Alkylsulfonylgruppen und Arylsulfonylgruppen besteht;

wobei alle obigen heterocyclischen Gruppen aus der Gruppe ausgewählt sind, die aus den in der Definition von $R^{6b}$ erwähnten Stickstoff-haltigen heterocyclischen Gruppen und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolyl-Gruppen besteht, umfassend das Umsetzen einer Verbindung, die durch die allgemeine Formel:

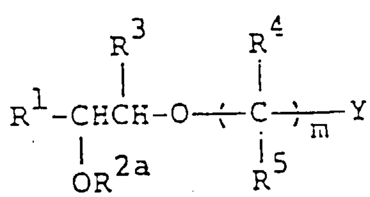

dargestellt wird, in der $R^1$, $R^{2a}$, $R^3$, $R^4$, $R^5$ und m die gleichen Bedeutungen, wie oben definiert, aufweisen und Y eine entfernbare Gruppe darstellt, mit einer Verbindung, die durch die folgende allgemeine Formel dargestellt wird, oder einem Salz derselben:

$$H\text{-}R^{6b}$$

in der $R^{6b}$ die gleiche Bedeutung, wie oben definiert, aufweist.

8. Verwendung eines 1,2-Ethandiol-Derivats, das durch die folgende allgemeine Formel dargestellt wird, oder eines Salzes desselben bei der Herstellung eines Arzneimittels zur Behandlung von zerebrovaskulärer Demenz, seniler Demenz, Alzheimer-Demenz, Folgezuständen von ischämischer Enzephalopathie oder zerebraler Apoplexie bei einem Patienten:

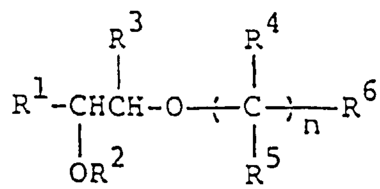

in der $R^1$, $R^2$, $R^3$, $R^4$, $R^5$, $R^6$ und n wie in Anspruch 1 definiert sind.

**9.** Verwendung eines 1,2-Ethandiol-Derivats oder eines Salzes desselben nach Anspruch 8, in dem $R^1$ eine substituierte oder unsubstituierte Naphthylgruppe darstellt; $R^2$ ein Wasserstoffatom oder eine Hydroxyl-Schutzgruppe darstellt; die nR4 gleich oder voneinander verschieden sind und Wasserstoffatome oder ($C_1$-$C_6$)-Alkylgruppen darstellen; die $nR^5$ Wasserstoffatome darstellen; und $R^6$ eine Ammoniogruppe oder eine substituierte oder unsubstituierte Amino- oder Stickstoff-haltige heterocyclische Gruppe darstellt, wobei die Stickstoff-haltige heterocyclische Gruppe aus der Gruppe ausgewählt ist, die aus Pyrrolyl-, Pyrrolidinyl-, Piperidinyl-, Piperazinyl-, Imidazolyl-, Pyrazolyl-, Pyridyl-, Pyrimidinyl-, Morpholinyl-, Chinolyl-, Chinolizinyl-, Tetrahydrochinolinyl-, Chinuklidinyl-, Thiazolyl- und Thiadiazolyl-Gruppen besteht.

**10.** Verwendung eines 1,2-Ethandiol-Derivats oder eines Salzes desselben nach Anspruch 8, in dem $R^1$ eine substituierte oder unsubstituierte Naphthyl-, Indanyl- oder Tetrahydronaphthyl-Gruppe darstellt; $R^6$ eine Ammoniogruppe oder eine substituierte oder unsubstituierte Amino- oder Stickstoff-haltige heterocyclische Gruppe darstellt, wobei die Stickstoff-haltige heterocyclische Gruppe aus der Gruppe ausgewählt ist, die aus Pyrrolinyl, Piperidyl, Piperazinyl, Imidazolyl, Pyridyl, Morpholinyl, Thiomorpholinyl, Tetrahydropyridyl, Chinuklidinyl und Tetrazolyl besteht, der Substituent an $R^1$ aus der Gruppe ausgewählt ist, die aus Halogenatomen, ($C_1$-$C_6$)-Alkylgruppen, ($C_2$-$C_6$)-Alkenylgruppen; Phenylgruppen, die gegebenenfalls durch ein Halogenatom oder eine ($C_1$-$C_6$)-Alkyl- oder ($C_1$-$C_6$)-Alkoxygruppe substituiert sind; Phenyl-($C_1$-$C_4$)-alkylgruppen; ($C_1$-$C_6$) -Alkoxygruppen, die gegebenenfalls durch eine Pyridyl-, Furyl- oder Thienylgruppe substituiert sein können; Phenyl-, ($C_1$-$C_4$)-Alkoxygruppen, die gegebenenfalls durch ein Halogenatom oder eine ($C_1$-$C_6$)-Alkyl-, Hydroxyl- oder ($C_1$-$C_6$)-Alkoxygruppe substituiert sein können; einer Phenoxygruppe; Phenylaminocarbonyloxy-Gruppen, die gegebenenfalls durch ein Halogenatom substituiert sein können; ($C_1$-$C_6$)-Alkylaminocarbonyloxy-Gruppen; ($C_1$-$C_6$)-Alkylthiogruppen; einer Phenylsulfonylaminogruppe; Aminogruppen, die gegebenenfalls durch eine ($C_1$-$C_6$)-Alkylgruppe substituiert sein können; einer Hydroxylgruppe; einer Nitrogruppe; einer Oxogruppe; Phenyl-($C_1$-$C_4$)-alkylthiogruppen; Phenyl-($C_1$-$C_4$)-alkylsulfonylgruppen; einer Thienylgruppe; einer Pyridylgruppe; und ($C_1$-$C_4$)-Alkylendioxygruppen besteht; der Substituent an $R^6$, wenn $R^6$ eine substituierte Aminogruppe ist, aus der Gruppe ausgewählt ist, die aus ($C_1$-$C_6$)-Alkylgruppen, welche gegebenenfalls durch eine Hydroxylgruppe substituiert sein können; ($C_1$-$C_6$)-Acylgruppen; Cycloalkylgruppen; Phenyl-($C_1$-$C_4$)-alkylgruppen; einer Pyrimidinyl-Gruppe; einer Pyridincarbonyl-Gruppe und einer Adamantylgruppe besteht; und der Substituent an $R^6$, wenn $R^6$ eine substituierte Stickstoff-haltige heterocyclische Gruppe ist, aus der Gruppe ausgewählt ist, die aus Halogenatomen; einer Hydroxylgruppe; ($C_1$-$C_6$)-Alkylgruppen, die gegebenenfalls durch eine Hydroxylgruppe substituiert sein können; einer Aminogruppe; ($C_1$-$C_6$)-Acylgruppen, die gegebenenfalls durch eine Pyrrolidinyl-Gruppe substituiert sein können; Phenyl-($C_1$-$C_4$)-alkylgruppen; Phenyl-($C_2$-$C_4$)-alkenylgruppen; Aroylgruppen, die durch ein Halogenatom substituiert sind; einer Pyridylgruppe und einer Oxogruppe besteht.

**11.** Verwendung eines 1,2-Ethandiol-Derivats oder eines Salzes desselben nach Anspruch 10, in dem $R^1$ eine Naphthyl-, Indanyl- oder Tetrahydronaphthyl-Gruppe darstellt, die gegebenenfalls durch ein Halogenatom oder eine ($C_1$-$C_6$)-Alkylgruppe substituiert sein kann; $R^2$ ein Wasserstoffatom darstellt; $R^3$ ein Wasserstoffatom darstellt; die $nR^4$ und $nR^5$ Wasserstoffatome darstellen; $R^6$ eine Morpholinyl-Gruppe, die an einem Kohlenstoffatom, das den Ring bildet, eine freie Valenz aufweist, eine Pyridylgruppe, eine Imidazolyl-Gruppe, eine durch eine Phenyl-$C^1$-$C^4$-alkylgruppe substituierte Piperazinyl-Gruppe oder eine Aminogruppe darstellt, die gegebenenfalls durch eine ($C_1$-$C_6$)-Alkylgruppe oder eine Cycloalkylgruppe substituiert sein kann; und n eine ganze Zahl von 1 bis 4 darstellt.

**12.** Verwendung eines 1,2-Ethandiol-Derivats oder eines Salzes desselben nach Anspruch 8, wobei es sich bei dem Derivat oder Salz um 2-(Imidazolylmethoxy)-1-(1-naphthyl)ethanol oder ein Salz desselben handelt.

**13.** Verwendung eines 1,2-Ethandiol-Derivats oder eines Salzes desselben nach Anspruch 8, wobei es sich bei dem Derivat oder Salz um 2-(1-Methyl-5-imidazolylmethoxy)-1-(1-naphthyl)ethanol oder ein Salz desselben handelt.

**14.** Ein die zerebrale Funktion verbesserndes Mittel, das ein 1,2-Ethandiol-Derivat umfaßt, welches durch die folgende allgemeine Formel dargestellt wird, oder ein Salz desselben:

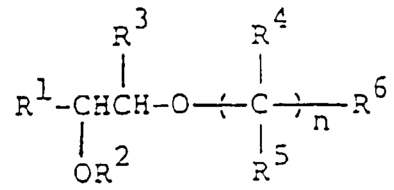

in der $R^1$,$R^2$,$R^3$,$R^4$,$R^5$, $R^6$ und n wie in Anspruch 1 definiert sind.

**Revendications**

**1.** Dérivé de 1,2-éthanediol représenté par la formule générale suivante, ou un sel de celui-ci:

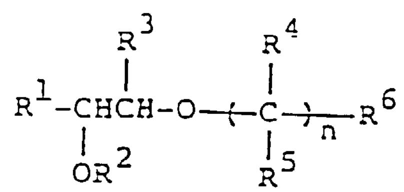

dans laquelle $R^1$ représente un groupe naphtyle, indanyle, indényle ou tétrahydronaphtyle, substitué ou non substitué; $R^2$ représente un atome d'hydrogène, un groupe alcoyle en $C_1$-$C_6$ ou un groupe protecteur d'hydroxyle; $R^3$ représente un atome d'hydrogène ou un groupe alcoyle en $C_1$-$C_6$; $nR^4$ et $nR^5$ sont identiques ou différents l'un de l'autre et représentent des atomes d'hydrogène ou des groupes alcoyle en $C_1$-$C_6$, $R^6$ représente un groupe ammonio ou un groupe amino ou hétérocyclique contenant de l'azote, substitués ou non substitués, ledit groupe hétérocyclique contenant de l'azote étant choisi dans le groupe consistant en les groupes pyrrolyle, pyrrolidinyle, pipéridyle, pipérazinyle, imidazolyle, pyrazolyle, pyridyle, tétrahydropyridyle, pyrimidinyle, morpholinyle, thiomorpholinyle, quinoléyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle; et n représente 0 ou un nombre entier de 1 à 6; dans lequel le substituant de $R^1$ est choisi dans le groupe consistant en les atomes d'halogène, un groupe aminé substitué ou non substitué, les groupes alcoyle en $C_1$-$C_6$, aryle, aryl-alcoyle en $C_1$-$C_4$, alcoxy en $C_1$-$C_6$, aryl-alcoxy en $C_1$-$C_4$, aryloxy, carbamoyloxy, alcoylthio en $C_1$-$C_6$, alcényle en $C_2$-$C_6$, alcényloxy en $C_2$-$C_6$, aryl-alcoylthio en $C_1$-$C_4$, aryl-alcoylsulfonyle en $C_1$-$C_6$, arylsulfonyle, alcoylsulfonylamino en $C_1$-$C_6$, arylsulfonylamino et hétérocyclique, les groupes aminés protégés, les groupes hydroxyles protégés et non protégés, le groupe nitro, le groupe oxo et les groupes alcoylènedioxy en $C_1$-$C_4$; les groupes substitués suivants: alcoyle en $C_1$-$C_6$, aryle, aryl-alcoyle en $C_1$-$C_4$, alcoxy en $C_1$-$C_6$, aryl-alcoxy en $C_1$-$C_4$, aryloxy, carbamoyloxy, alcoylthio en $C_1$-$C_6$, alcényle en $C_2$-$C_6$, alcényloxy en $C_2$-$C_6$, aryl-alcoylthio en $C_1$-$C_4$, aryl-alcoylsulfonyle en $C_1$-$C_6$, arylsulfonyle, alcoylsulfonylamino en $C_1$-$C_6$, arylsulfonylamino ou hétérocyclique en tant que substituant de $R^1$ et le groupe hétérocyclique substitué contenant de l'azote en tant que $R^6$ portant chacun au moins un substituant choisi dans le groupe consistant en les atomes d'halogène, les groupes hydroxyles protégés ou non protégés, les groupes aminés protégés ou non protégés, les groupes carboxyliques protégés ou non protégés, les groupes alcoyles en $C_1$-$C_6$ non substitués, les groupes alcoyles en $C_1$-$C_6$ substitués par un groupe hydroxyle protégé ou non protégé, les groupes aryles non substitués ou substitués par un halogène, les groupes aroyles non substitués ou substitués par un halogène, les groupes alcoxy en $C_1$-$C_6$ non substitués, les groupes alcoxy en $C_1$-$C_6$ substitués par un groupe alcoxy en $C_1$-$C_6$, les groupes acyles en $C_1$-$C_6$, les groupes aryl-acoyles en $C_1$-$C_4$, les groupes aryl-alcényles en $C_2$-$C_6$, les groupes hétérocycliques, les groupes hétérocycle-CO-, le groupe oxo, les groupes alcoylsulfonyles en $C_1$-$C_6$ et les groupes arylsulfonyles; et le groupe aminé substitué en tant que substituant de $R^1$ et le groupe aminé substitué en tant que $R^6$ portant chacun au moins un substituant choisi dans le groupe consistant en les groupes hydroxyles protégés ou non protégés, les groupes alcoyles en $C_1$-$C_6$ non substitués, les groupes alcoyles en $C_1$-$C_6$ substitués par un groupe carboxylique ou hydroxyle protégé ou non protégé, les groupes cycloalcoyles, les groupes aryles, les groupes acyles en $C_1$-$C_6$, les groupes aryl-alcoyles en $C_1$-$C_4$, les groupes hétérocycliques, les groupes hétérocycle-CO- non substitués ou substitués par un groupe oxo, le groupe adamantyle, les groupes alcoylsulfonyles en $C_1$-$C_6$ et les groupes arylsulfonyles; les groupes hétérocycliques ci-dessus étant tous choisis dans le groupe consistant en les groupes hétérocycliques contenant de l'azote

mentionnés à la définition de $R^6$ et les groupes furyle, thiényle, benzothiényle, pyranyle, isobenzofuranyle, oxazolyle, benzofuranyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-l-thianaphtyle, 2,3-dihydrobenzofuranyle, benzo[b]dioxanyle, imidazo[2,3-a]pyridyle, benzo[b]pipérazinyle, chroményle, isothiazolyle, isoxazolyle, oxadiazolyle, pyridazinyle, isoindolyle et isoquinoléyle.

**2.** Dérivé de 1,2-éthanediol ou un sel de celui-ci selon la revendication 1, dans lequel $R^1$ représente un groupe naphtyle substitué ou non substitué; $R^2$ représente un atome d'hydrogène ou un groupe protecteur d'hydroxyle; les $nR^4$ sont identiques ou différents l'un de l'autre et représentent des atomes d'hydrogène ou des groupes alcoyles en $C_1$-$C_6$; les $nR^5$ représentent des atomes d'hydrogène; et $R^6$ représente un groupe ammonio ou un groupe aminé ou hétérocyclique contenant de l'azote, substitués ou non substitués, le groupe hétérocyclique contenant de l'azote étant choisi dans le groupe consistant en les groupes pyrrolyle, pyrrolidinyle, pipéridinyle, pipérazinyle, imidazolyle, pyrazolyle, pyridyle, pyrimidinyle, morpholinyle, quinoléyle, quinolizinyle, tétrahydroquinolinyle, quinuclidinyle, thiazolyle et thiadiazolyle.

**3.** Dérivé de 1,2-éthanediol ou un sel de celui-ci selon la revendication 1, dans lequel $R^1$ représente un groupe naphtyle, indanyle ou tétrahydronaphtyle qui peuvent être éventuellement substitués par un atome d'halogène ou un groupe alcoyle en $C_1$-$C_6$; $R^2$ représente un atome d'hydrogène; $R^3$ représente un atome d'hydrogène; les $nR^4$ et les $nR^5$ représentent des atomes d'hydrogène; $R^6$ représente un groupe morpholinyle dont un atome de carbone du cycle présente une valence libre, un groupe pyridyle, un groupe imidazolyle, un groupe pipérazinyle substitué par un groupe phényl-alcoyle en $C_1$-$C_4$ ou un groupe aminé qui peut être éventuellement substitué par un groupe alcoyle en $C_1$-$C_6$ ou cycloalcoyle; et n représente un nombre entier de 1 à 4.

**4.** Le 2-(imidazolylméthoxy)-1-(1-naphtyl)éthanol ou un sel de celui-ci.

**5.** Le 2-(1-méthyl-5-imidazolylméthoxy)-1-(1-naphtyl)éthanol ou un sel de celui-ci.

**6.** Procédé de préparation d'un dérivé de 1,2-éthanediol représenté par la formule générale suivante, ou d'un sel de celui-ci:

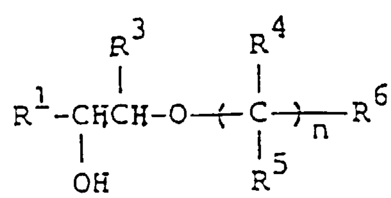

formule dans laquelle $R^1$, $R^3$, $R^4$, $R^5$, $R^6$ et n sont tels que définis à la revendication 1, selon lequel on fait réagir un composé représenté par la formule générale:

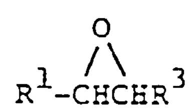

dans laquelle $R^1$ et $R^3$ ont la même la signification qu'indiqué à la revendication 1, avec un composé représenté par la formule générale suivante ou un sel de celui-ci:

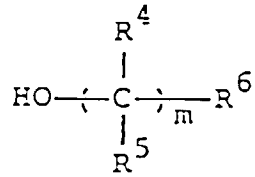

formule dans laquelle les $mR^4$ et les $mR^5$ sont identiques ou différents l'un de l'autre et représentent des atomes d'hydrogène ou des groupes alcoyles inférieurs, $R^6$ a la même signification qu'indiqué à la revendication 1 et m représente un nombre entier de 1 à 6, ou avec un composé représenté par la formule générale suivante ou un sel de celui-ci

$$HO\text{-}R^{6a}$$

formule dans laquelle $R^{6a}$ représente le même groupe hétérocyclique contenant de l'azote, substitué ou non substitué, que dans la définition de $R^6$, étant entendu que le groupe hétérocyclique présente une valence libre sur un atome de carbone qui fait partie de l'hétérocycle.

**7.** Procédé de préparation d'un dérivé de 1,2-éthanediol représenté par la formule générale suivante ou d'un sel de celui-ci:

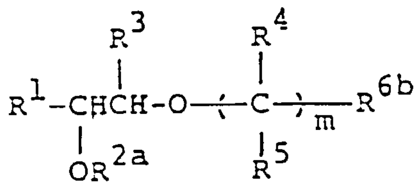

formule dans laquelle $R^1$et $R^3$ sont tels que défini à la revendication 1, $R^{2a}$ représente un groupe protecteur d'hydroxyle, les $mR^4$ et les $mR^5$ sont identiques ou différents l'un de l'autre et représentent des atomes d'hydrogène ou des groupes alcoyles en $C_1$-$C_6$; $R^{6b}$ représente un groupe aminé ou hétérocyclique contenant de l'azote, substitués ou non substitués, le groupe hétérocyclique contenant de l'azote, présentant une valence libre sur un atome de carbone qui fait partie de l'hétérocyclique et étant choisi dans le groupe consistant en pyrrolyle, pyrrolidinyle, pipéridyle, pipérazinyle, imidazolyle, pyrazolyle, pyridyle, tétrahydropyridyle, pyrimidinyle, morpholinyle, thiomorpholinyle, quinoléyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle; et m représente un nombre entier de 1 à 6, dans lequel le groupe hétérocyclique substitué contenant de l'azote, en tant que $R^{6b}$, porte au moins un substituant choisi dans le groupe consistant en les atomes d'halogène, les groupes hydroxyles protégés ou non protégés, les groupes aminés protégés ou non protégés, les groupes carboxyliques protégés ou non protégés, les groupes alcoyles en $C_1$-$C_6$ non substitués, les groupes alcoyles en $C_1$-$C_6$ substitués par un groupe hydroxyle protégé ou non protégé, les groupes aryles non substitués ou substitués par un halogène, les groupes aroyles non substitués ou substitués par un halogène, les groupes alcoxy en $C_1$-$C_6$ non substitués, les groupes alcoxy en $C_1$-$C_6$ substitués par un groupe alcoxy en $C_1$-$C_6$, les groupes acyles en $C_1$-$C_6$, les groupes aryl-acoyles en $C_1$-$C_4$, les groupes aryl-alcényles en $C_2$-$C_6$, les groupes hétérocycliques, les groupes hétérocycle-Co-, le groupe oxo, les groupes alcoylsulfonyles en $C_1$-$C_6$ et les groupes arylsulfonyles; et le groupe aminé substitué en tant que $R^{6b}$ porte au moins un substituant choisi dans le groupe consistant en les groupes hydroxyles protégés ou non protégés, les groupes alcoyles en $C_1$-$C_6$ non substitués, les groupes alcoyles en $C_1$-$C_6$ substitués par un groupe carboxylique ou hydroxyle protégé ou non protégé, les groupes cycloalcoyles, les groupes aryles, les groupes acyles en $C_1$-$C_6$, les groupes aryl-alcoyles en $C_1$-$C_4$, les groupes hétérocycliques, les groupes hétérocycle-CO- non substitués ou substitués par un groupe oxo, le groupe adamantyle, les groupes alcoylsulfonyles en $C_1$-$C_6$ et les groupes arylsulfonyles; les groupes hétérocycliques ci-dessus étant tous choisis dans le groupe consistant en les groupes hétérocycliques contenant de l'azote mentionnés à la définition de $R^{6b}$ et les groupes furyle, thiényle, benzothiényle, pyranyle, isobenzofuranyle, oxazolyle, benzofuranyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofuranyle, benzo-[b]dioxanyle, imidazo [2,3-a]pyridyle, benzo[b]pipérazinyle, chroményle, isothiazolyle, isoxazolyle, oxadiazolyle, pyridazinyle, isoindolyle et isoquinoléyle, selon lequel on fait réagir un composé représenté par la formule générale:

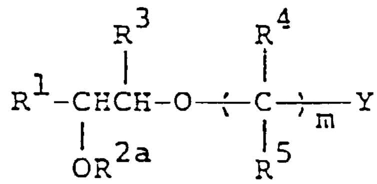

dans laquelle $R^1$, $R^{2a}$, $R^3$, $R^4$, $R^5$ et m ont la même signification qu'indiqué ci-dessus et Y représente un groupe amovible, avec un composé représenté par la formule générale suivante ou un sel de celui-ci:

$$H\text{-}R^{6b}$$

formule dans laquelle $R^{6b}$ a la même signification qu'indiqué ci-dessus.

**8.** Utilisation d'un dérivé de 1,2-éthanediol représenté par la formule générale suivante ou d'un sel de celui-ci pour préparer un médicament destiné à traiter un patient atteint de démence cérébro-vasculaire, de démence sénile, de la maladie d'Alzheimer, de suites d'encéphalopathie ou d'apoplexie cérébrale:

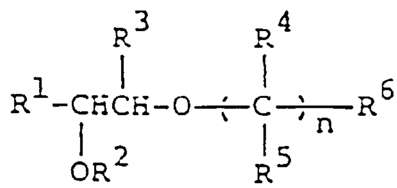

formule dans laquelle $R^1$, $R^2$, $R^3$, $R^4$, $R^5$, $R^6$ et n sont tels que défini à la revendication 1.

**9.** Utilisation d'un dérivé de 1,2-éthanediol ou d'un sel de celui-ci selon la revendication 8, dans laquelle $R^1$ représente un groupe naphtyle substitué ou non substitué; $R^2$ représente un atome d'hydrogène ou un groupe protecteur d'hydroxyle; les $nR^4$ sont identiques ou différents l'un de l'autre et représentent des atomes d'hydrogène ou des groupes alcoyles en $C_1$-$C_6$; les $nR^5$ représentent des atomes d'hydrogène; et $R^6$ représente un groupe ammonio ou un groupe aminé ou hétérocyclique contenant de l'azote, substitués ou non substitués, le groupe hétérocyclique contenant de l'azote étant choisi dans le groupe consistant en les groupes pyrrolyle, pyrrolidinyle, pipéridinyle, pipérazinyle, imidazolyle, pyrazolyle, pyridyle, pyrimidinyle, morpholinyle, quinoléyle, quinolizinyle, tétrahydroquinolinyle, quinuclidinyle, thiazolyle et thiadiazolyle.

**10.** Utilisation d'un dérivé de 1,2-éthanediol ou d'un sel de celui-ci selon la revendication 8, dans laquelle $R^1$ représente un groupe naphtyle, indanyle ou tétrahydronaphtyle, substitué ou non substitué; $R^6$ représente un groupe ammonio ou un groupe aminé ou hétérocyclique contenant de l'azote, substitués ou non substitués, le groupe hétérocyclique contenant de l'azote étant choisi dans le groupe consistant en les groupes pyrrolinyle, pipéridyle, pipérazinyle, imidazolyle, pyridyle, morpholinyle, thiomorpholinyle, tétrahydropyridyle, quinuclidinyle et tétrazolyle, le substituant de $R^1$ est choisi dans le groupe consistant en les atomes d'halogène; les groupes alcoyles en $C_1$-$C_6$; les groupes alcényles en $C_2$-$C_6$; les groupes phényles qui peuvent être substitués éventuellement par un atome d'halogène ou un groupe alcoyle en $C_1$-$C_6$ ou alcoxy en $C_1$-$C_6$; les groupes phényl-alcoyles en $C_1$-$C_4$; les groupes alcoxy en $C_1$-$C_6$ qui peuvent être substitués éventuellement par un groupe pyridyle, furyle ou thiényle; les groupes phényl-alcoxy en $C_1$-$C_4$ qui peuvent être substitués éventuellement par un atome d'halogène ou par un groupe alcoyle en $C_1$-$C_6$, hydroxyle ou alcoxy en $C_1$-$C_6$; le groupe phénoxy; le groupe phénylaminocarbonyloxy qui peut être substitué éventuellement par un atome d'halogène; les groupes alcoylaminocarbonyloxy en $C_1$-$C_6$; les groupes alcoylthio en $C_1$-$C_6$; le groupe phénylsulfonylamino; les groupes aminés qui peuvent être substitués éventuellement par un groupe alcoyle en $C_1$-$C_6$; le groupe hydroxyle; le groupe nitro; le groupe oxo; les groupes phénylalcoylthio en $C_1$-$C_4$; les groupes phényl-alcoylsulfonyles en $C_1$-$C_4$; le groupe thiényle; le groupe pyridyle; et les groupes alcoylènedioxy en $C_1$-$C_4$; le substituant de $R^6$, lorsque $R^6$ est un groupe aminé substitué, étant choisi dans le groupe consistant en les groupes alcoyles en $C_1$-$C_6$ qui peuvent être substitués éventuellement par un groupe hydroxyle; les groupes acyles en $C_1$-$C_6$; les groupes cycloalcoyles; les groupes phényl-alcoyles en $C_1$-$C_4$; un groupe pyrimidinyle; un groupe pyridinecarbonyle et un groupe adamantyle; et le substituant de $R^6$, lorsque $R^6$

est un groupe hétérocyclique substitué contenant de l'azote, est choisi dans le groupe consistant en les atomes d'halogène; le groupe hydroxyle; les groupes alcoyles en $C_1$-$C_6$ qui peuvent être substitutés éventuellement par un groupe hydroxyle; un groupe aminé; les groupes acyles en $C_1$-$C_6$ qui peuvent être substitués éventuellement par un groupe pyrrolidinyle, les groupes phényl-alcoyles en $C_1$-$C_4$; les groupes phényl-alcényles en $C_2$-$C_4$; les groupes aroyles qui peuvent être substitués par un atome d'halogène; un groupe pyridyle et un groupe oxo.

**11.** Utilisation d'un dérivé de 1,2-éthanediol ou d'un sel de celui-ci selon la revendication 1, dans laquelle $R^1$ représente un groupe naphtyle, indanyle ou tétrahydronaphtyle qui peuvent être éventuellement substitués par un atome d'halogène ou un groupe alcoyle en $C_1$-$C_6$; $R^2$ représente un atome d'hydrogène; $R^3$ représente un atome d'hydrogène; les $nR^4$ et les $nR^5$ représentent des atomes d'hydrogène; $R^6$ représente un groupe morpholinyle dont un atome de carbone du cycle présente une valence libre, un groupe pyridyle, un groupe imidazolyle, un groupe pipérazinyle substitué par un groupe phényl-alcoyle en $C_1$-$C_4$ ou un groupe aminé qui peut être éventuellement substitué par un groupe alcoyle en $C_1$-$C_6$ ou cycloalcoyle; et n représente un nombre entier de 1 à 4.

**12.** Utilisation d'un dérivé de 1,2-éthanediol ou d'un sel de celui-ci selon la revendication 8, dans laquelle le dérivé ou sel est le 2-(imidazolylméthoxy)-1-(1-naphtyl)éthanol ou un sel de celui-ci.

**13.** Utilisation d'un dérivé de 1,2-éthanediol ou d'un sel de celui-ci selon la revendication 8, dans laquelle le dérivé ou sel est le 2-(1-méthyl-5-imidazolylméthoxy)-1-(1-naphtyl)éthanol ou un sel de celui-ci.

**14.** Agent d'amélioration des fonctions cérébrales qui comprend un dérivé de 1,2-éthanediol représenté par la formule générale suivante ou un sel de celui-ci:

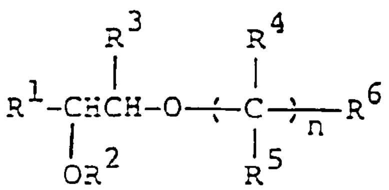

formule dans laquelle $R^1$, $R^2$, $R^3$, $R^4$, $R^5$, $R^6$ et n sont tels que définis à la revendication 1.